# EUROPEAN PATENT SPECIFICATION

(11) **EP 0 815 221 B1**
(45) Date of publication and mention of the grant of the patent: **22.10.2003**
(21) Application number: 96902810.9
(22) Date of filing: 22.02.1996
(51) Int. Cl.: C07K 14/475, A61K 38/18

(54) **A NOVEL GROWTH FACTOR AND A GENETIC SEQUENCE ENCODING SAME**
EIN NEUARTIGER WACHSTUMFAKTOR UND EINE GENETISCHE SEQUENZ DIE FÜR DIESEN KODIERT
NOUVEAU FACTEUR DE CROISSANCE ET SEQUENCE GENETIQUE CODANT CE FACTEUR

(30) Priority: 02.03.1995 AU PN145795; 20.11.1995 AU PN664795; 22.12.1995 AU PN727495
(43) Date of publication of application: 07.01.1998
(73) Proprietor: AMRAD Operations Pty.,Ltd., Richmond, Victoria 3121 (AU)
(72) Inventor: HAYWARD, Nicholas, Kim, Paddington, QLD 4064 (AU); WEBER, Gunther, Kew, VIC 3101 (AU); GRIMMOND, Sean, Taringa, QLD 4068 (AU); NORDENSKJOLD, Magnus, S-161 37 Stockholm (SE); LARSSON, Catharina, S-113 44 Stockholm (SE)
(74) Representative: Mallalieu, Catherine Louise
(86) International application number: AU9600094
(87) International publication number: WO96027007

(56) References cited:
- WO-A-96/26736
- AU-A- 5 657 490
- AU-A- 6 079 890
- AU-A- 7 394 194
- US-A- 5 073 492
- MAGLIONE D. ET AL.: "Isolation of a human placenta cDNA coding for a protein related to the vascular permeability factor" PROCEEDINGS OF THE NATIONAL ACADEMY OF SCIENCES USA, vol. 88, October 1991, pages 9267-9271, XP002080555
- MAGLIONE D. ET AL.: "Two alternative mRNAs coding for the angiogenic factor, placenta growth factor (PlGF), are transcribed from a single gene of chromosome 14" ONCOGENE, vol. 8, no. 4, April 1993, pages 925-931, XP002080556
- BIOCHEMICAL AND BIOPHYSICAL RESEARCH COMMUNICATIONS, (1992), Vol. 183, No. 3, WEINDEL K. et al., "Aids-Associated Kaposi's Sarcoma Cells in Culture Express Vascular Endothelial Growth Factor", pages 1167-1174.
- BIOCHEMICAL AND BIOPHYSICAL RESEARCH COMMUNICATIONS, (1989), Vol. 165, No. 3, EDMUND TISCHER et al., "Vascular Endothelial Growth Factor: A New Member of the Platelet-Derived Growth Factor Gene Family", pages 1198-1206.
- JOURNAL OF VIROLOGY, 1994, Vol. 68, No. 1, DAVID J. LYTTLE et al., "Homologs of Vascular Endothelial Growth Factor Are Encoded by the Pox Virus Orf Virus", pg. 84-92.
- METHODS IN ENZYMOLOGY, 1991, Vol. 198, FERRARA NAPOLIANA et al., "Purification and Cloning of Vascular Endothelial Growth Factor Secreted by Pituitary Folliculostellate Cells", pg. 391-405.
- THE JOURNAL OF BIOLOGICAL CHEMISTRY, 1991, Vol. 266, No. 18, EDMUND TISCHER et al., "The Human Gene for Vascular Endothelial Growth Factor", pg. 11947-11954.
- BIOCHEMICAL AND BIOPHYSICAL RESEARCH COMMUNICATIONS, 1996, Vol. 220, No. 1, LAGERCRANTZ J. et al., "Expression of the VEGF-Related Factor Gene in Pre- and Postnatal Mouse", pg. 147-52.
- BIOCHIMICA ET BIOPHYSICA ACTA, 1995, Vol. 1260, No. 2, SHARMA HARI S. et al., "Nucleotide Sequence and Expression of the Porcine Vascular Endothelial Growth Factor", pg. 235-9.
- DEVELOPMENT, 1992, Vol. 114, BREIER G. et al., "Expression of Vascular Endothelial Growth Factor During Embryonic Angiogenesis and Endothelial Cell Differentiation", pg. 521-532.
- MOLECULAR ENDOCRINOLOGY, 1991, Vol. 5, No. 12, HOUCK KEITH A. et al., "The Vascular Endothelial Growth Factor Family: Identification of a Fourth Molecular Species and Characterization of Alternative Splicing of RNA", pg. 1806-1814.

## Description

The present invention relates generally to an isolated molecule having vascular endothelial growth factor-like properties and to a genetic sequence encoding same. The molecule will be useful in the development of a range of therapeutics and diagnostics useful in the treatment, prophylaxis and/or diagnosis of conditions requiring enhanced or diminished vasculature and/or vascular permeability. The molecule of the present invention is also a useful effector of primary and central neurons and is capable of inducing astroglial proliferation.

Bibliographic details of the publications referred to by author in this specification are collected at the end of the description. Sequence Identity Numbers (SEQ ID NOs.) for the nucleotide and amino acid sequences referred to in the specification are defined following the bibliography.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Vascular endothelial growth factor (hereinafter referred to as "VEGF"), also known as vasoactive permeability factor, is a secreted, covalently linked homodimeric glycoprotein that specifically activates endothelial tissues (Senger *et al*., 1993). A range of functions have been attributed to VEGF such as its involvement in normal angiogensis including formation of the corpus luteum (Yan *et al.,* 1993) and placental development (Sharkey *et al.,* 1993), regulation of vascular permeability (Senger *et al.,* 1993), inflammatory angiogenesis (Sunderkotter *et al*., 1994) and autotransplantation (Dissen *et al.,* 1994) and human diseases such as tumour promoting angiogenesis (Folkman & Shing, 1992), rheumatoid arthritis (Koch *et al*., 1994) and diabetes related retinopathy (Folkman & Shing, 1992).

VEGF is, therefore, an important molecule making it a potentially valuable target for research into therapeutics, prophylactics and diagnostic agents based on VEGF or its activities. There is also a need to identify homologues or otherwise related molecules for use as an alternative to VEGF or in conjunction with VEGF.

In work leading up to the present invention, the inventors sought the multiple endocrine neoplasia type I susceptibility gene (MEN1). Surprisingly, the inventors discovered that a genetic sequence excluded as a candidate for the MEN1 gene was nevertheless a new growth factor having some similarity to VEGF. Furthermore, the growth factor of the present invention is an effector molecule for primary and central neurons.

Accordingly, one aspect of the present invention provides an isolated polypeptide which exhibits at least one of the following properties:
(i) an ability to induce proliferation of vascular endothelial cells;
(ii) an ability to interact with *flt*-1/*flk*-1 family of receptors; and/or
(iii) an ability to induce cell migration, cell survival and/or an increase in intracellular levels of alkaline phosphatase;
wherein said polypeptide comprises:
(a) an amino acid sequence encoded by the nucleotide sequence as set forth in SEQ ID NO:3 or a nucleotide sequence which hybridizes over the full length of SEQ ID NO:3 or its complementary form under high stringency conditions of 0.1-1 x SSC/0.1% w/w SDS at 60°C for 1-3 hours; or
(b) an amino acid sequence as set forth in SEQ ID NO:4 or a truncated form of said polypeptide which exhibits at least one of properties (I)-(iii).

By "biologically isolated" is meant that the molecule has undergone at least one step of purification from a biological source. Preferably, the molecule is also biologically pure meaning that a composition comprises at least about 20%, more preferably at least about 40%, still more preferably at least about 65%, even still more preferably at least about 80-90% or greater of the molecule as determined by weight, activity or other convenient means, relative to other compounds in the composition. Most preferably, the molecule is sequencably pure.

Another preferred aspect of the present invention provides the molecule in recombinant form.

The present invention also contemplates genomic or partial genome clones encoding a proteinaceous molecule of the present invention.

The amino acid sequence set forth in SEQ ID NO:2 corresponds to human VEGF (referred to herein as "VEGF₁₆₅"). Accordingly, the molecule of the present invention is VEGF-like or is a homologue of VEGF but comprises an amino acid sequence which is similar but non-identical to the amino sequence of VEGF. Although the present invention is exemplified using a human VEGF-like molecule, this is done with the understanding that the instant invention contemplates the homologous molecule and encoding sequence from other mammals such as livestock animals (e.g. sheep, pigs, horses and cows), companion animals (e.g. dogs and cats) and laboratory test animals (e.g. mice, rats, rabbits and guinea pigs) as well as non-mammals such as birds (e.g. poultry birds), fish and reptiles. In a most preferred embodiment, the VEGF-like molecule is of human origin and encoded by a gene located at chromosome 11q13. The present invention extends, therefore, to the genomic sequence or part thereof encoding the subject VEGF-like molecule.

Preferably, the percentage similarity is at least about 30%, more preferably at least about 40%, still more preferably at least about 50%, still even more preferably at least about 60-70%, yet even more preferably at least about 80-95% to all or part of the amino acid sequence set forth in SEQ ID NO:2.

In one aspect, the VEGF-like molecule of the presant invention comprises a sequence of amino acids as set forth in SEQ ID NO:4 or is a part, fragment, derivative or analogue thereof. Reference herein to derivatives also includes splice variants. Accordingly, the present invention extends to splice variants of SOM175. Examples of splice variants contemplated by the present invention include but are not limited to variants with an amino acid sequence substantially as set forth in at least one of SEQ ID NO:8 and/or SEQ ID NO:10 or mutants or derivatives or further splice variants thereof.

Thus, still a further aspect of the present invention contemplates a peptide fragment corresponding to a portion of the amino acid sequence set forth in SEQ ID NO:4 or a splice variant thereof such as set forth in SEQ ID NO:8 or SEQ ID NO.10 or a chemical equivalent thereof. The biologically isolated or recombinant molecule of the present invention may be naturally glycosylated or may comprise an altered glycosylation pattern depending on the cells from which it is isolated or synthesised. For example, if produced by recombinant means in prokaryotic organisms, the molecule would be non-glycosylated. The molecule may be a full length, naturally occurring form or may be a truncated or otherwise derivatised form.

Yet another aspect of the present invention is directed to a nucleic acid molecule encoding the VEGF-like molecule herein described. More particularly, the present invention provides an isolated nucleic acid molecule comprising the nucleotide sequence set forth in SEQ ID NO:3 or a nucleotide sequence which hybridizes over the full length of the nucleotide sequence set forth in SEQ ID NO:3 or its complementary form under high stringency conditions of 0.1-1 x SSC/0.1 % w/w SDS at 60°C for 1-3 hours.

For the purposes of defining the level of stringency, reference can conveniently be made to Sambrook *et al* (1989) at pages 9.47-9.51 which is herein incorporated by reference where the washing steps disclosed are considered high stringency. A low stringency is defined herein as being in 4-6X SSC/0.1-0.5% w/v SDS at 37-45°C for 2-3 hours. Depending on the source and concentration of nucleic acid involved in the hybridisation, alternative conditions of stringency may be employed such as. medium stringent conditions which are considered herein to be 1-4X SSC/0.25-0.5% w/v SDS at ≥ 45°C for 2-3 hours or high stringent-conditions considered herein to be 0.1-1X SSC/0.1% w/v SDS at 60°C for 1-3 hours.

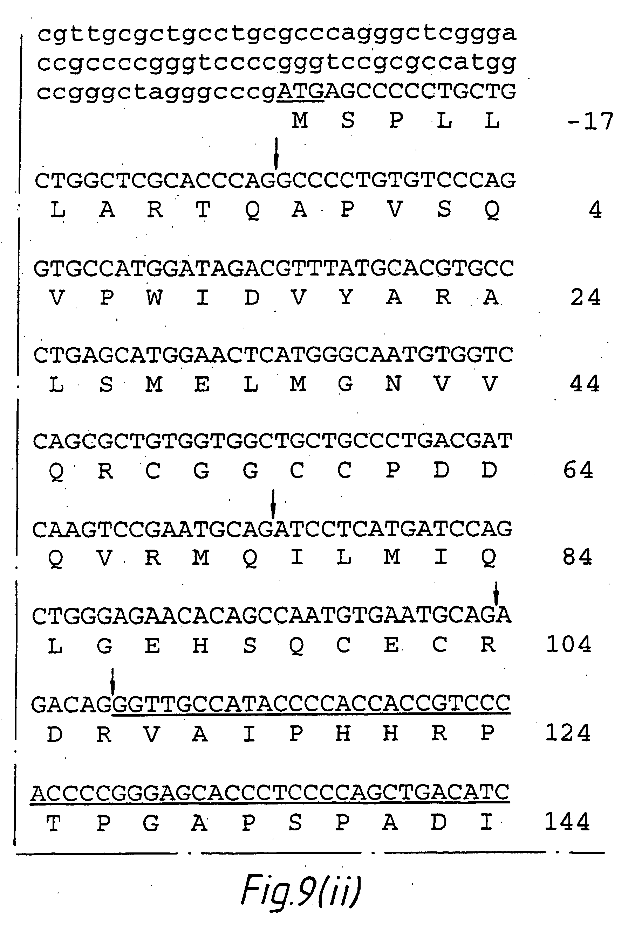

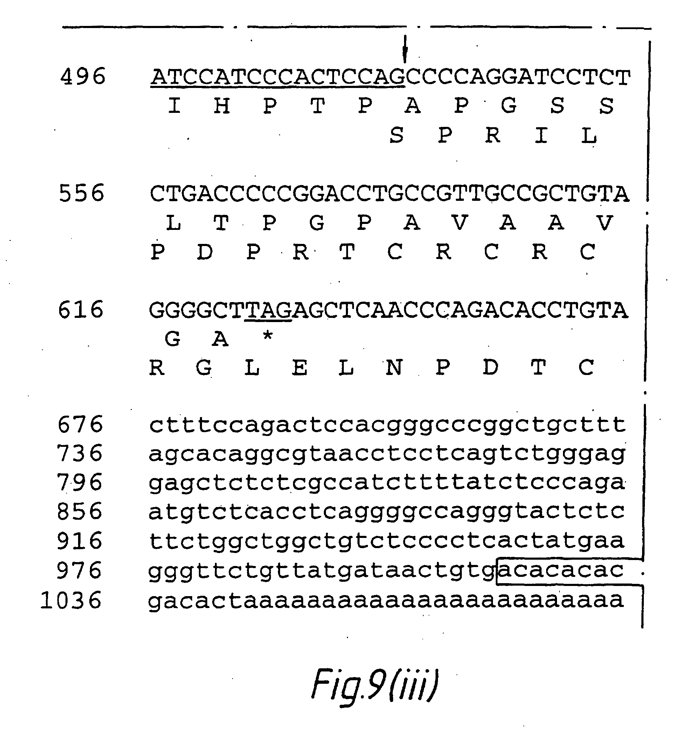

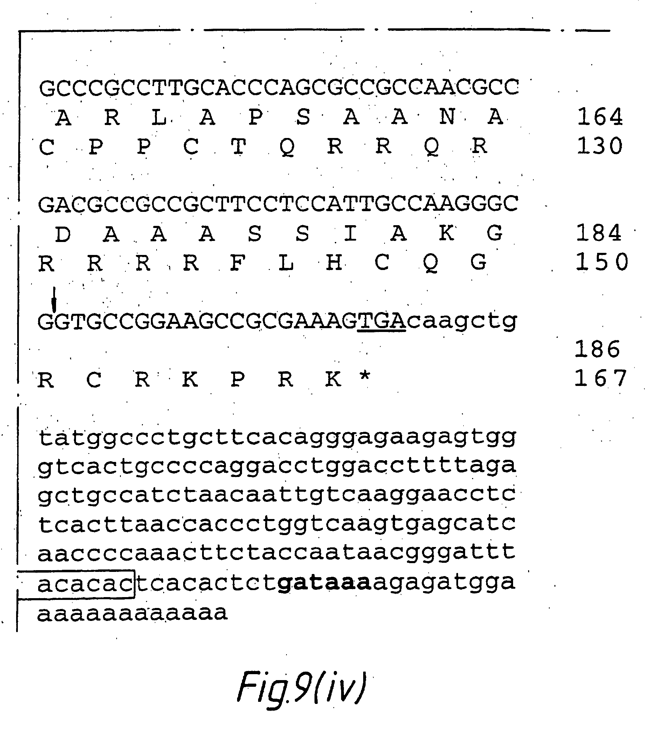

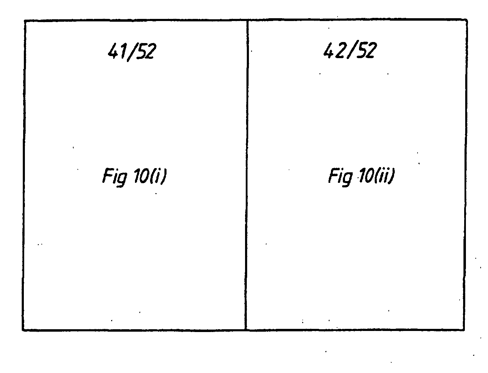

The present invention is further directed to the marine homologue of human VEGF (referred to herein as "SOM175"). The SOM175 has approximately 85% identity and 92% conservation of amino acid residues over the entire coding region compared to human VEGF. The SOM175 is encoded by a nucleic acid molecule comprising a nucleotide sequence substantially as set forth in Figure 9.

The VEGF-Iike molecule of the present invention will be useful in the development of a range of therapeutic and/or diagnostic applications alone or in combination with other molecules such as VEGF. The present invention extends, therefore, to pharmaceutical compositions comprising the VEGF-like molecule or parts, fragments, derivatives, homologues or analogues thereof together with one or more phannaccudcally acceptable carriers and/or diluents. In addition, the present invention extends to ribozymes and antisense molecules based on SEQ ID NO:3 as well as neutralizing antibodies to the VEGF-like module. Such molecules may be useful in ameliorating the effects of, for example, over expression of VEGF-like genes leading to angiogenesis or vascularization of tumours.

The present invention also contemplates antibodies to the VEGF-like molecule of the present invention or nucleic acid probes to a gene encoding the VEGF-like molecule of the present invention which are useful as diagnostic agents.

According to a further aspect of the present invention, there is provided an isolated neutralizing antibody to a polypeptide of the invention.

In another aspect of the present invention there is provided a composition comprising a polypeptide of the invention and one or more phannaceutically acceptable carriers and/or diluents.

In another aspect of the present invention there is provided a host comprising a nucleic acid molecule according to the present invention.

In yet another aspect of the present invention there is provided a vector comprising a nucleic acid molecule according to the present invention.

In a further aspect of the present invention there is provided use of a polypeptide which exhibits at least one of the following properties:
(i) an ability to induce proliferation of vascular endomelial cells;
(ii) an ability to interact with *flt-*1/*flk*-1 family of receptors; and/or
(iii) an ability to induce cell migration, cell survival and/or an increase in intracellular levels of alkaline phosphatase;
wherein said polypeptide comprises:
(a) an amino acid sequence encoded by the nucleotide sequence as set forth in SEQ ID NO: 1, 3, 5, 7, or 9 or a nucleotide sequence capable of hybridizing to SEQ ID NO: 1,3,5,7 or 9 or its complementary form under high stringency conditions of 0.1-1 x SSC/0.1% w/w SDS at 60°C for 1-3 hours; or
(b) an amino acid sequence as set forth in SEQ ID NO: 2, 4, 6, 8 or 10 or an amino acid sequence having at least 70% similarity thereto, or a truncated form of said polypeptide,
for preparation of a medicament for inducing astroglial proliferation in a mammal.

Preferably, the recombinant proteinaceous molecule comprises the amino acid sequence set forth in SEQ ID NO:4 or SEQ ID NO:6.

The present invention is further described by reference to the following non-limiting Figures and/or Examples.

In the Figures:
**Figure 1** Nucleotide sequence [SEQ ID NO:1] and corresponding amino acid sequence [SEQ ID NO:2] of VEGF₁₆₅.
**Figure 2** Nucleotide sequence [SEQ ID NO:3] and corresponding amino acid sequence [SEQ ID NO:4] of SOM175.
**Figure 3** Results of BLAST search with SOM175 protein sequence.
**Figure 4** BESTFIT alignment of VEGF cDNA and SOM175 cDNA.
**Figure 5** Multiple alignment of VEGF₁₆₅ with SOM175 and its splice variants at the nucleotide level.
**Figure 6** Multiple alignment of VEGF₁₆₅ with SOM175 and its splice variants at the amino acid level.
**Figure 7** Diagrammatic representation of SOM175 and its splice variants.
**Figure 8(a)** Diagrammatic representation of genomic structure of human SOM175 genomic showing exon/intron map.
**Figure 8(b)** Diagrammatic representation of genomic structure of human SOM175 showing exon/intron boundries.
**Figure 9** Nucleotide and predicted peptide sequences derived from mSOM175 cDNA clones. Numbering of nucleotides are given on the left, starting from the A of the initiation codon. Amino acids are numbered on the right, starting from the first residue of the predicted mature protein after the putative signal peptide has been removed. The alternately spliced region is double underlined and the resulting peptide sequence from each mRNA is included. A potential polyadenylation signal is indicated in boldface. Start and stop codons of mSOM175₁₆₇ and mSOM175₁₈₆ are underlined and a polymorphic AC repeat in the 3' UTR is indicated by a stippled box. The positions of intron/exons boundaries are indicated by arrowheads.
**Figure 10** BESTFIT alignments of human and murine VRF protein isoforms. A:mSOM175₁₆₇ and hSOM175₁₆₇. B: mSOM175₁₈₆ and hSOM175₁₈₆ from the point where the sequences diverge from the respective 167 amino acid isoforms. Amino acid identitites are marked with vertical bars and conserved amino acids with colons. An arrow marks the predicted signal peptide cleavage site of human and mouse SOM175.
**Figure 11** BESTFIT alignment of mSOM175₁₆₇ and mVEGF₁₈₈ (Breier *et al*, 1992) peptide sequences. An arrow marks the signal peptide cleavage site of m VEGF. Identical amino acids are indicated by vertical bars and conservative substitutions by colons. Numbering of amino acids is as described in the legend to Figure 9.
**Figure 12** Comparison of gene structure between SOM175 (a generic SOM175 gene is shown since the intron/exon organisation of the mouse and human homologues is almost identical) and other members of the human VEGF/PIGF/PDGF gene family. Exons are represented by boxes. Protein coding regions and untranslated regions are shown by filled and open sections respectively. The hatched region in SOM175 indicates the additional 3' UTR sequence formed by alternate splicing of the SOM175₁₈₆ isoform. Potential alternate splice products of each gene are shown.

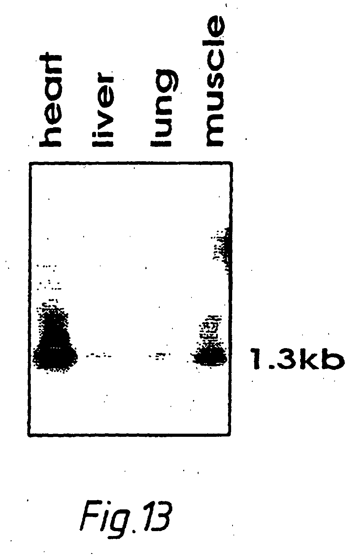

**Figure 13** Autoradiogram of a Northern blot of total RNA from various adult mouse tissues (as indicated) hybridised with an mVRF cDNA clone. A major transcript of 1.3 kb was detected in all samples.

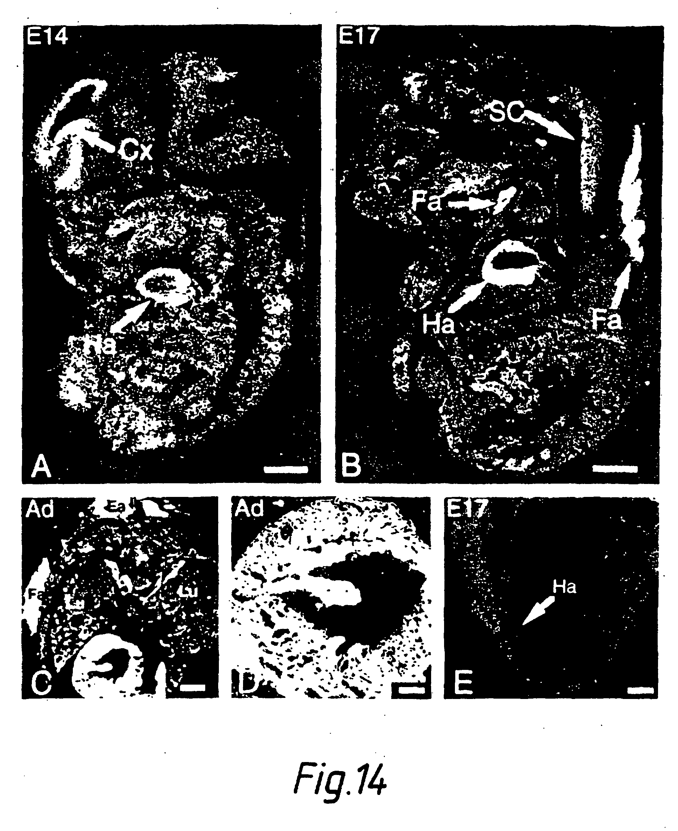

**Figure 14** Film autoradiographs (A-C) and dark-field micrographs (D-E) illustrating the expression pattern of mVRF and mRNA in the mouse. In the E14 mouse embryo (A) positive signals are present over the developing heart (Ha) and cerebral cortex (Cx). A low background signal is also present over other tissues in the section. In the E17 embryo (B) and the heart (Ha) is clearly visible due to a strong hybridisation signal. An equally strong signal is present over brown adipose tissue (Fa) in the back and around the thoracic cage. A moderate hybridisation signal is present over the spinal cord (SC) and the tongue (T). The background signal is reduced compared with the E14 embryo. In the young adult mouse (C-D), positive signals are present over the heart (Ha) and adipose tissue (Fa) around the thoracic cage, while, for example, the lungs (Lu) are unlabeled). The hybridisation signal over the heart is evenly distributed over the entire left ventricle, including papillary muscles (D). In the E17 heart hybridised with an excess of cold probe, no positive signal is present (E). Scale bars = 0.5 mm (A), 1.2 mm (B), 1 mm (C), 0.3 mm (D), 0.1 mm (E).

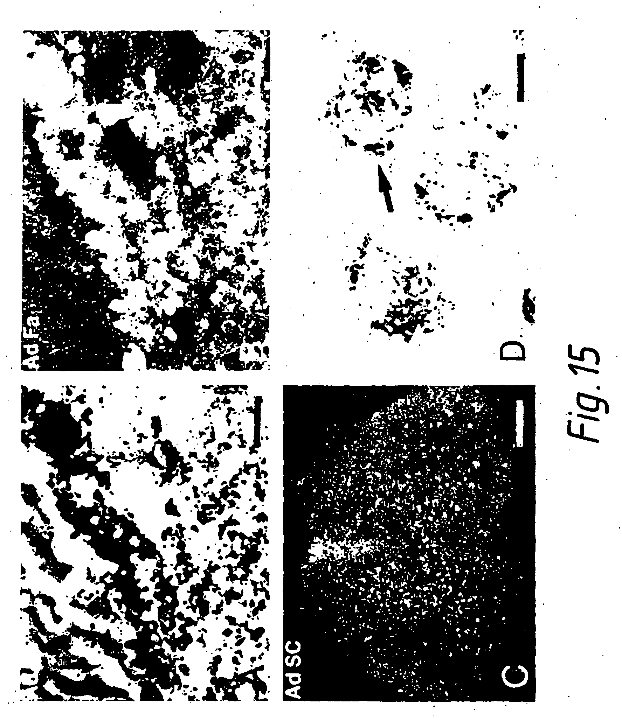

**Figure 15** Dark - (A and C) and bright-field (B and D) micrographs showing mSOM175 mRNA expression in mouse adipose tissue (A-B) and spinal cord (C-D). A strong hybridisation signal is present over fat (A), as shown by the strong labeling in Sudan black stained sections (B). A weak signal is present also in skeletal muscle (M in A-B). In the adult spinal cord (C) the mSOM175 probes gave a neuronal staining pattern over the gray matter. Toloudine counterstaining showing that motoneurons in the ventral horn (D), interneurons in the deep part of the dorsal horn and around the central canal (not shown) where largely positive for mSOM175 mRNA. Scale bars = 0.1 mm (A), 0.1 mm (B), 0.25 mm (C), 0.015 mm (D).
**Figure 16** Effect of VEGF on embryonic day 8 (E8) chick sensory neurons as determined by % survival, % neurite outgrowth and average neurite length (µm).
**Figure 17** Effects of VEGF and SOM175 on chick glia. Tested were CNS glial, peripheral glia and CNS oligodendrocytes.
**Figure 18** Effect of various SOM175 proteins on mouse astroglial cells. ■ ³H (cpm)
   1. FGF-2 (10 ng/ml) positive control
   2. SOMΔX6* 1 ng/ml
   3. SOMΔX6 10 ng/ml
   4. SOMΔX6 100 ng/ml
   5. SOMΔX6 1000 ng/ml
   6. SOMΔX6 1000 ng/ml, no heparin
   7. SOMX6** 1 ng/ml
   8. SOMX6 10 ng/ml
   9. SOMX6 100 ng/ml
   10. SOMX6 1000 ng/ml
   11. SOMX6 1000 ng/ml, no heparin
**Figure 19** Effect of various SOM175 proteins on mouse oligodenroglial cells. ■ ³H (cpm)
   1. FGF-2 (10 ng/ml) positive control
   2. SOMΔX6* 1 ng/ml
   3. SOMΔX6 10 ng/ml
   4. SOMΔX6 100 ng/ml
   5. SOMΔX6 1000 ng/ml
   6. SOMΔX6 1000 ng/ml, no heparin
   7. SOMX6** 1 ng/ml
   8. SOMX6 10 ng/ml
   9. SOMX6 100 ng/ml
   10. SOMX6 1000 ng/ml
   11. SOMX6 1000 ng/ml, no heparin
**Figure 20** Effect of various SOM175 proteins on mouse forebrain neurons. ■ % survival
   1. FGF-2 (10 ng/ml) positive control
   2. SOMΔX6* 1 ng/ml
   3. SOMΔX6 10 ng/ml
   4. SOMΔX6 100 ng/ml
   5. SOMΔX6 1000 ng/ml
   6. SOMΔX6 1000 ng/ml, no heparin
   7. SOMX6** 1 ng/ml
   8. SOMX6 10 ng/ml
   9. SOMX6 100 ng/ml
   10. SOMX6 1000 ng/ml
   11. SOMX6 1000 ng/ml, no heparin

**TABLE 1**

| SUMMARY OF SEQUENCE IDENTITY NUMBERS | |
|---|---|
| SEQ ID NO:1 | Nucleotide sequence of VEGF₁₆₅ |
| SEQ ID NO:2 | Amino acid sequence of VEGF₁₆₅ |
| SEQ ID NO:3 | Nucleotide sequence of SOM175 (VEGF-like molecules) |
| SEQ ID NO:4 | Amino acid sequence of SOM175 |
| SEQ ID NO:5 | Nucleotide sequence of SOM175 absent exon 6 |
| SEQ ID NO:6 | Amino acid sequence of SOM175 absent exon 6 |
| SEQ ID NO:7 | Nucleotide sequence of SOM175 absent exon 6 and exon 7 |
| SEQ ID NO:8 | Amino acid sequence of SOM175 absent exon 6 and exon 7 |
| SEQ ID NO:9 | Nucleotide sequence of SOM175 absent exon 4 |
| SEQ ID NO:10 | Amino acid sequence of SOM175 absent exon 4 |
| SEQ ID NO:11 | Oligonucleotide |
| SEQ ID NO:12 | Oligonucleotide |
| SEQ ID NO:13 | Oligonucleotide |
| SEQ ID NO:14 | Oligonucleotide |

*This refers to SOM175 absent exon 6;
** This refers to SOM175.
*This refers to SOM175 absent exon 6;
** This refers to SOM175.

### EXAMPLE 1

### Human cDNA clones

The original SOM175 cDNA was isolated by screening a human foetal brain library (λzapII, Stratagene) with the cosmid D11S750 (Larsson *et al*, 1992). The plasmid was excised "*in vivo*" and a single 1.1kb cDNA was obtained. Three independent SOM175 cDNAs clones were also isolated from a human foetal spleen library (Strategane, Unizap) using the above-mentioned SOM175 insert as a probe. Three clones were obtained: SOM175-4A, -5A and -6A. SOM175-5A is an alternately spliced clone with exon 4 being absent (SOM175-e4). These library screens were performed using hybridisation conditions recommended by the manufacturer of the library (Stratagene) and random primed insert of SOM175.

Two partial human SOM175 cDNAs have also isolated from a λGT11 human melanoma cell line A2058 library (Clontech) cDNA library screens were performed using hybridisation conditions described by Church and Gilbert, 1984). In each case, the probe was generated by random priming of a PCR product derived from SOM175 (18f-700r).

### Mouse cDNA Clones

Human SOM175 was also used to screen a mouse neonatal whole brain cDNA library (Unizap, Stratagene). Four non-chimeric clones were isolated: M175-A, B, C, D. All clones were partial cDNAs and M175-C contained several introns. Three of these cDNAs lacked the exon 6.

Another clone referred to as M1 was completely sequenced and was found to contain the full open reading frame plus part of the 5'utr and total 3'utr.

### EXAMPLE 2

### DNA SEQUENCE ANALYSIS

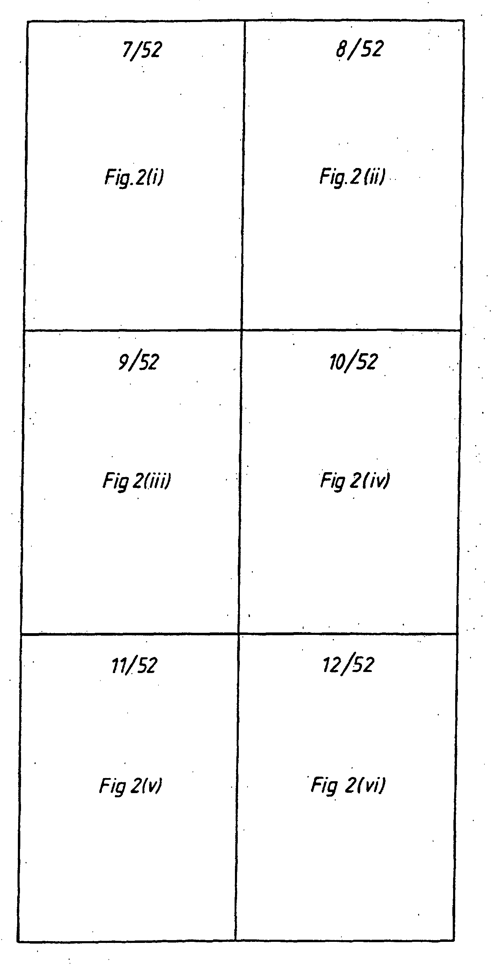

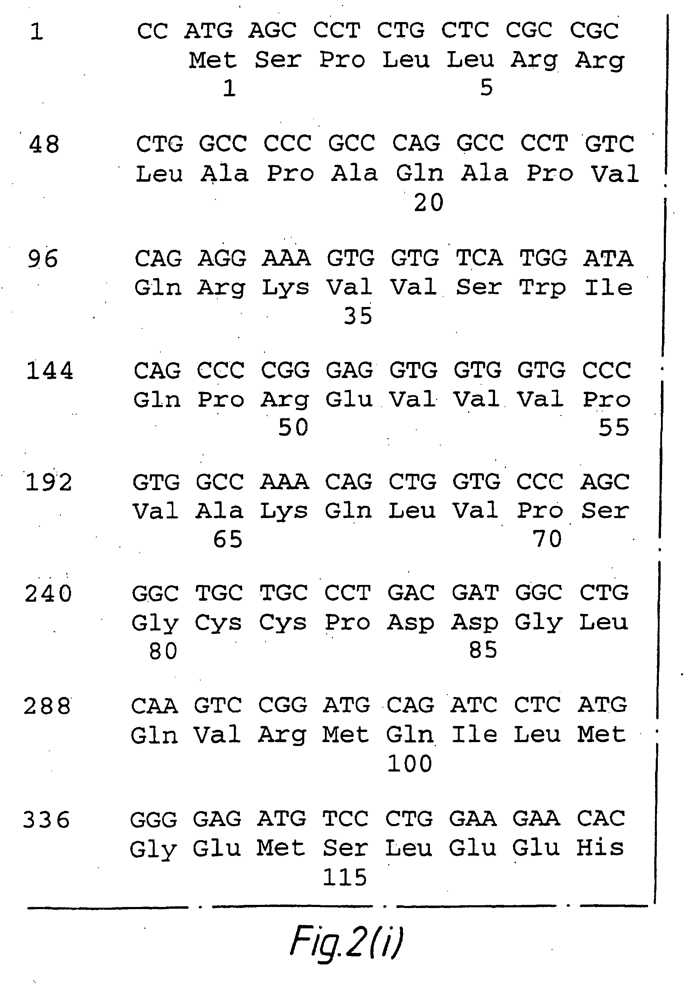

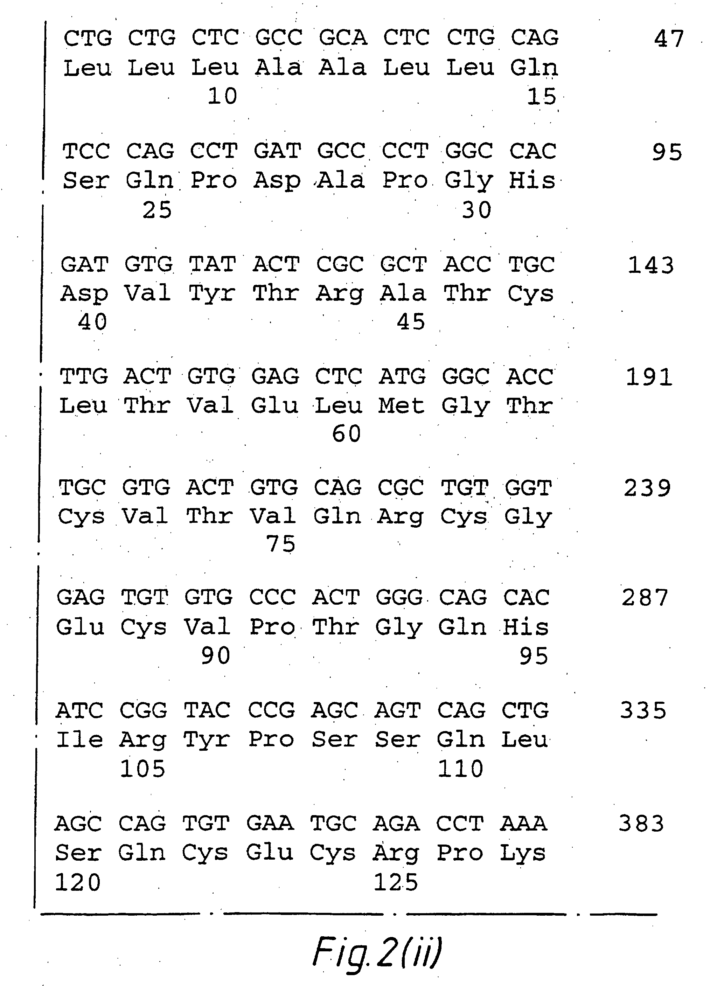

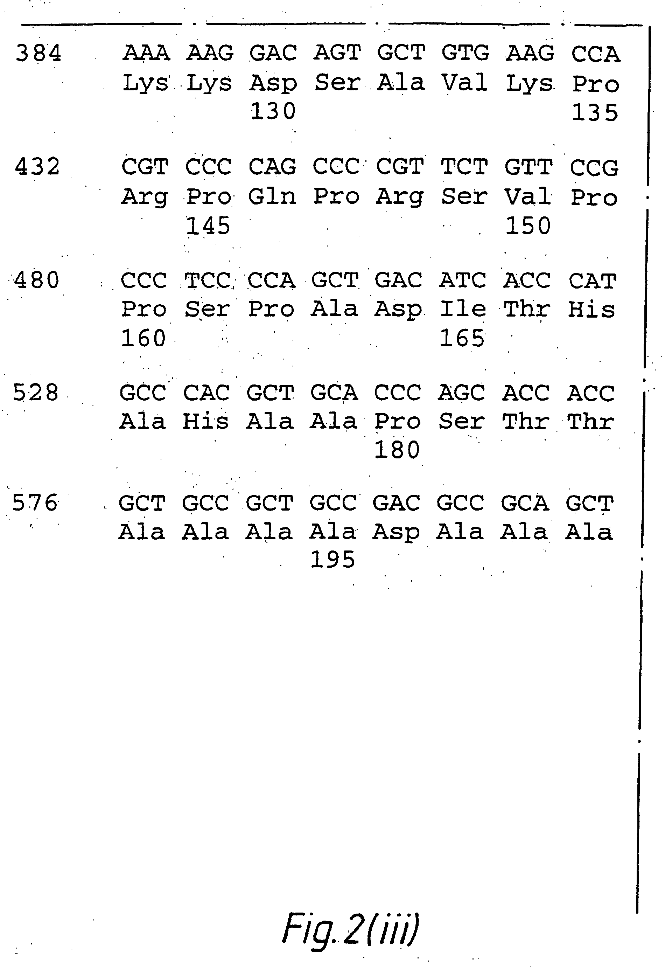

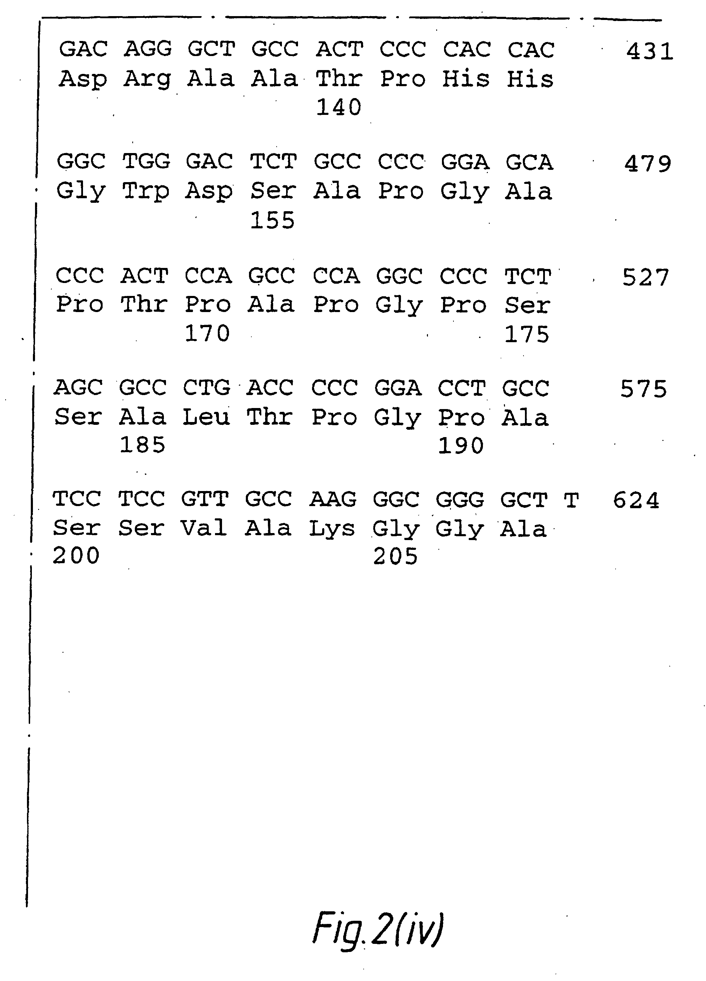

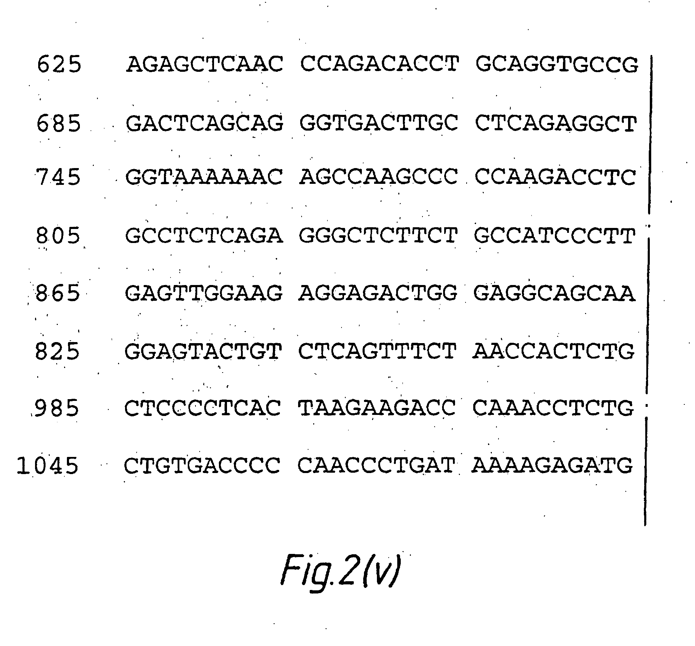

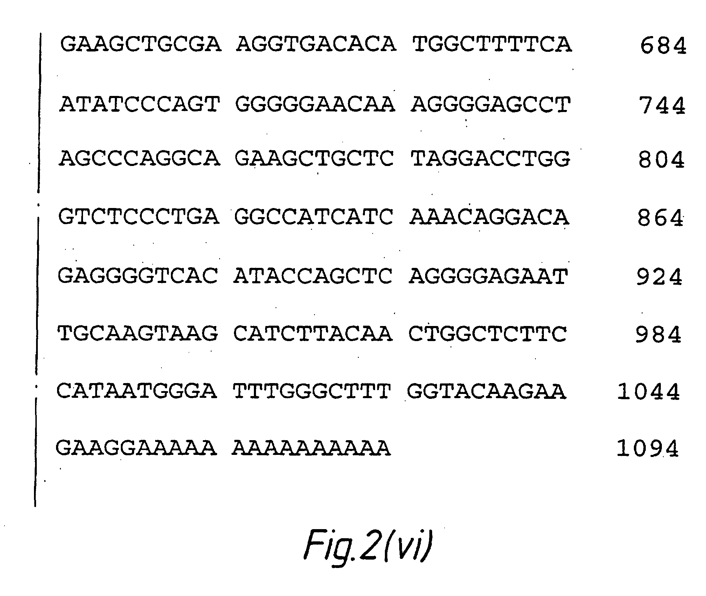

The entire sequence of the cDNA clone (SOM175) was compiled and is shown in Figure 2 with its corresponding amino acid sequence. This sequence was screened for open reading frames using the MAP program (GCG, University of Wisconsin). A single open reading frame of 672bp was observed (see Figure 2). There appears to be little 5' untranslated sequences (2bp). The 3' untranslated region appears to be complete as it includes a poly-adenylation signal and poly-A tail.

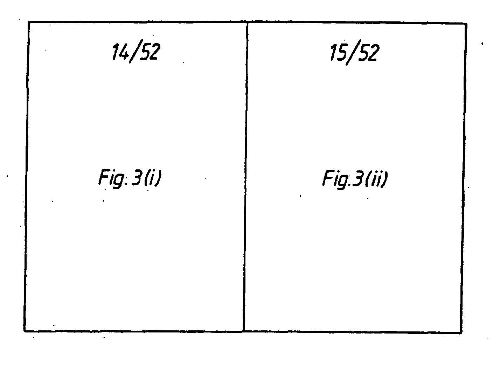

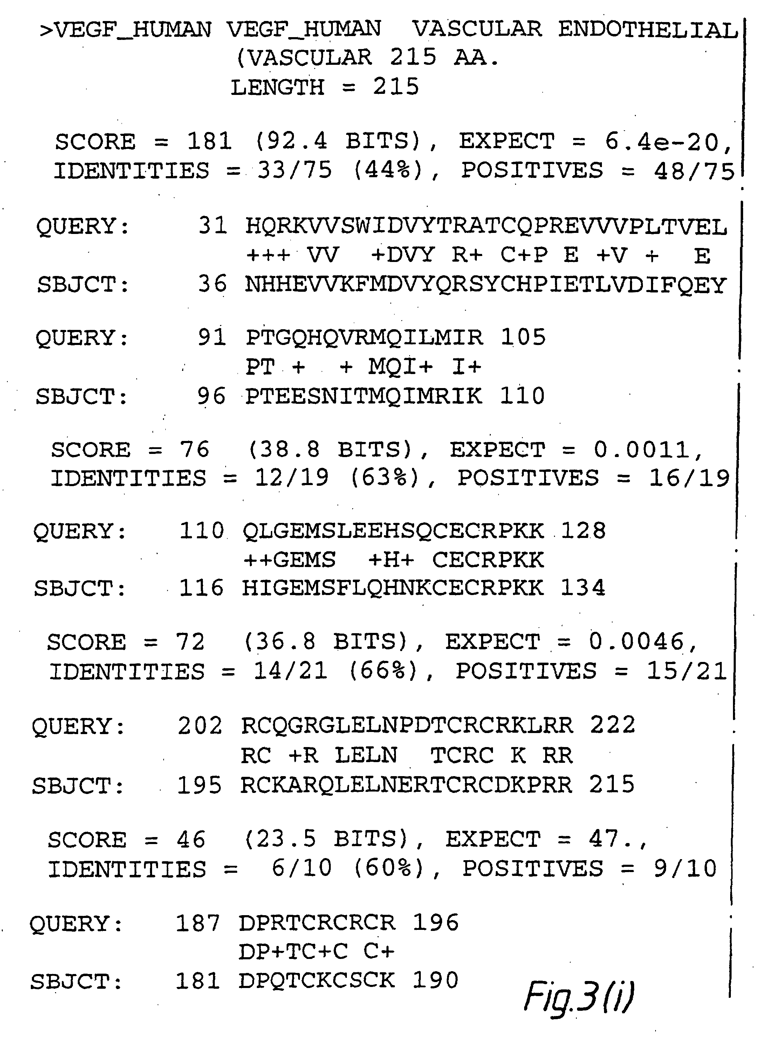

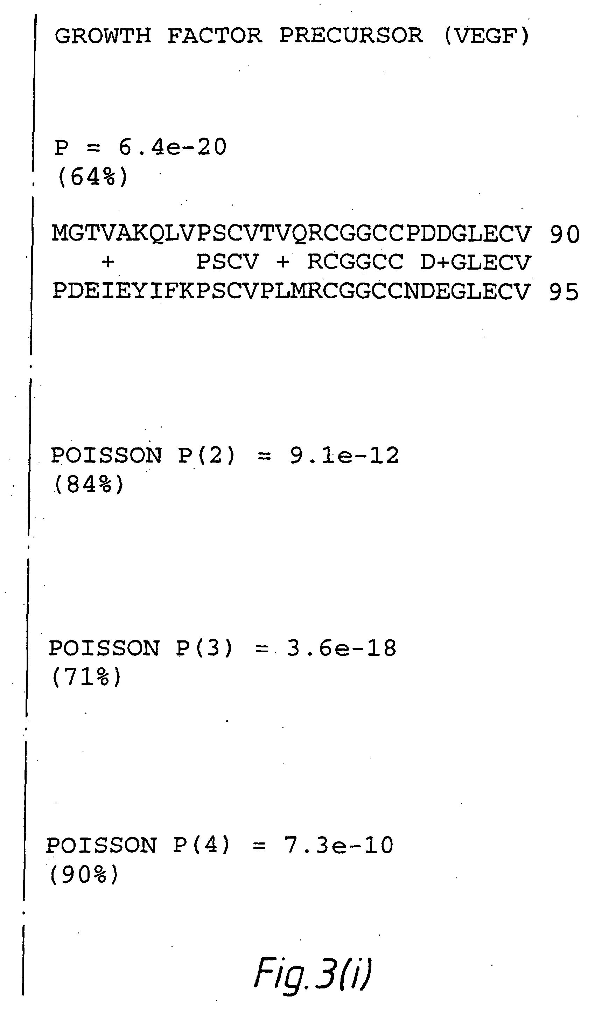

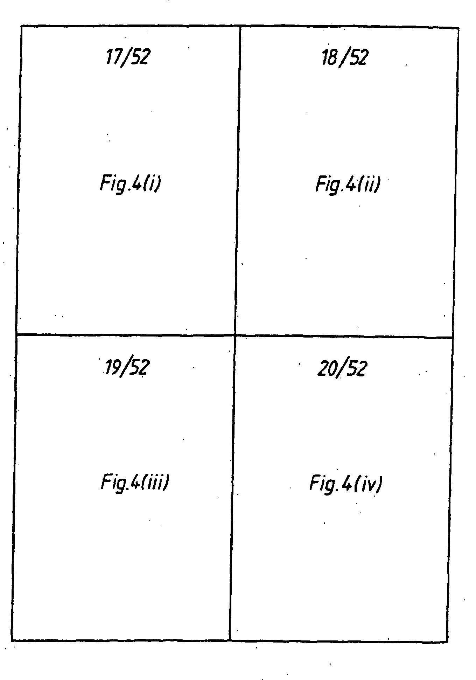

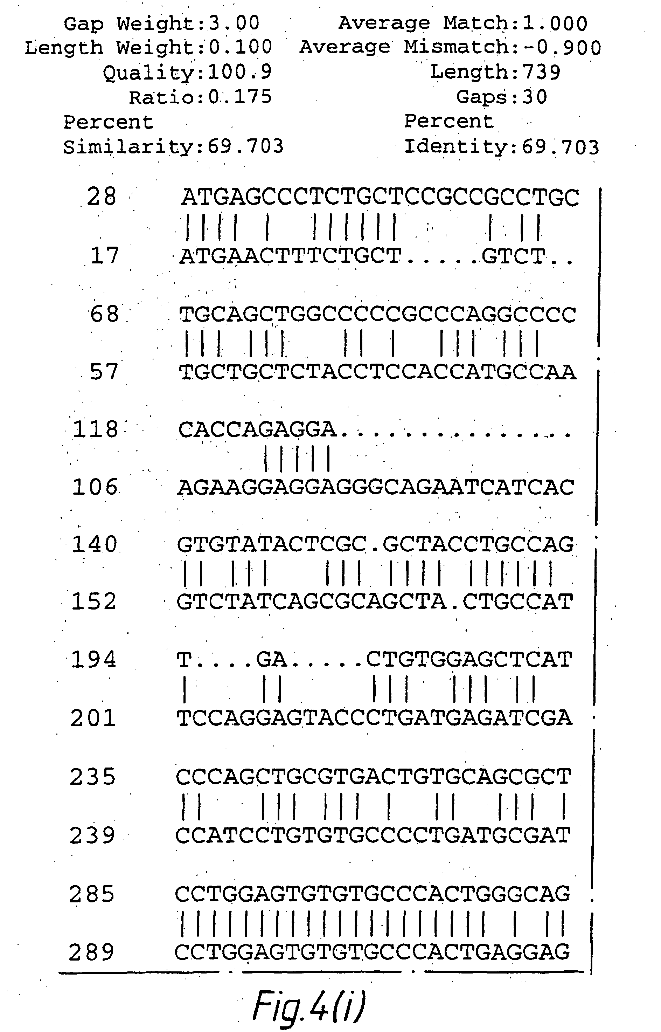

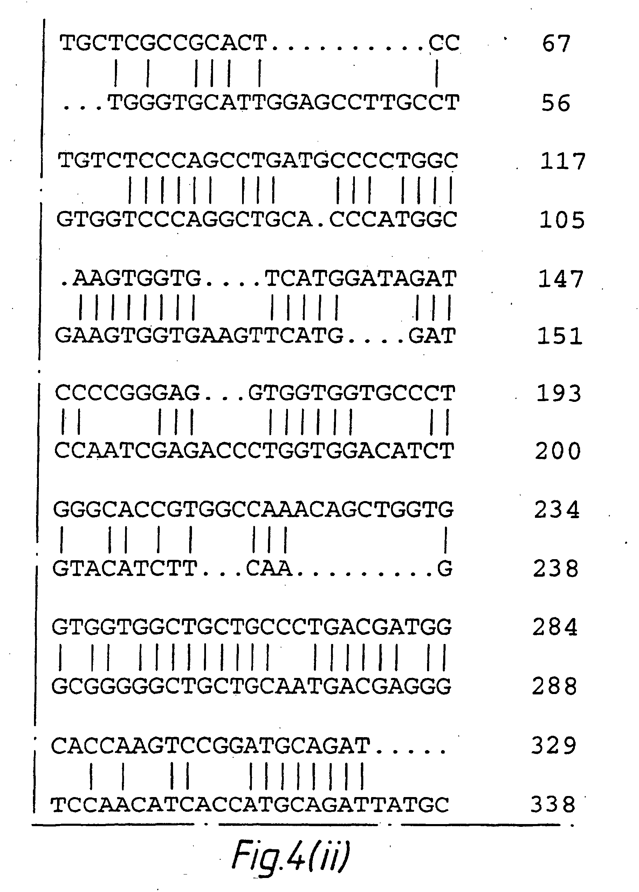

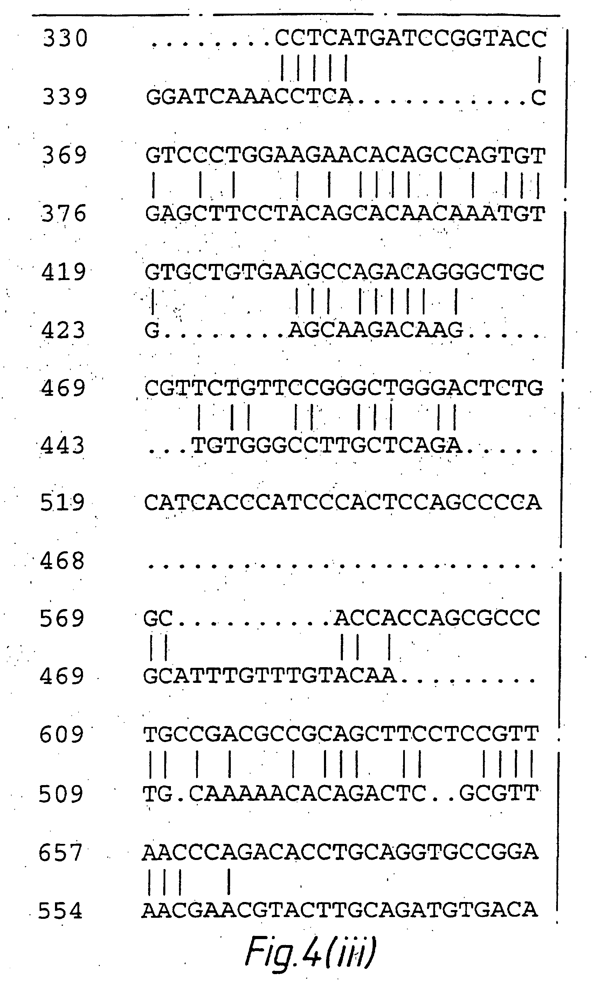

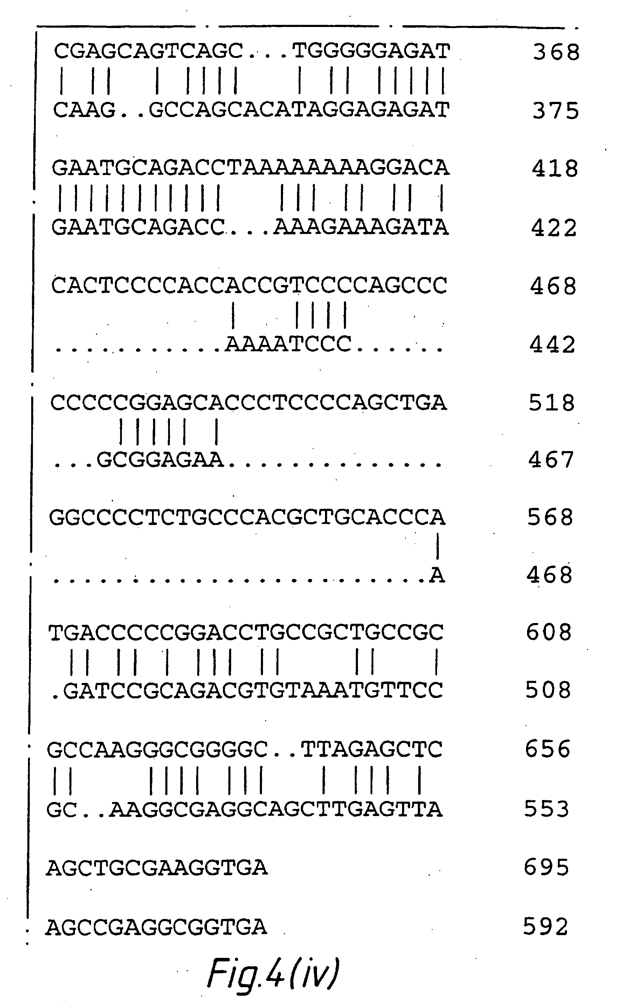

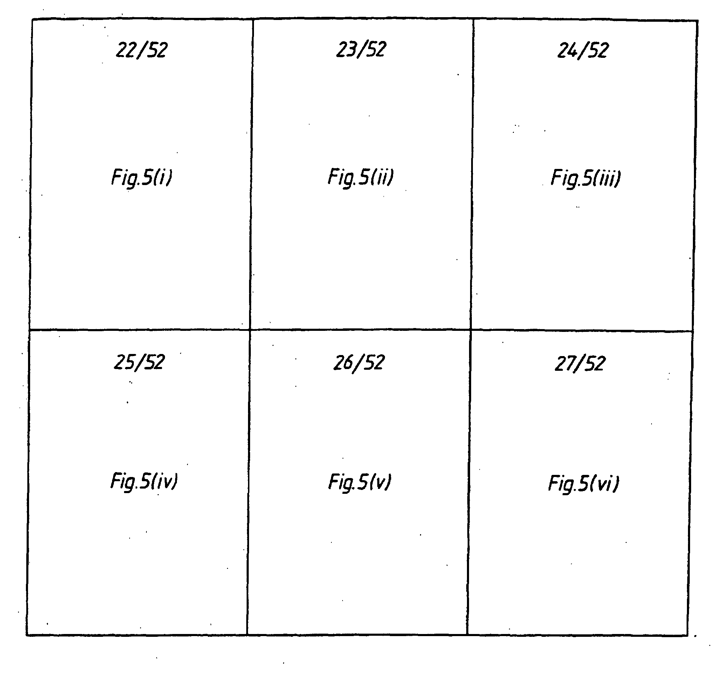

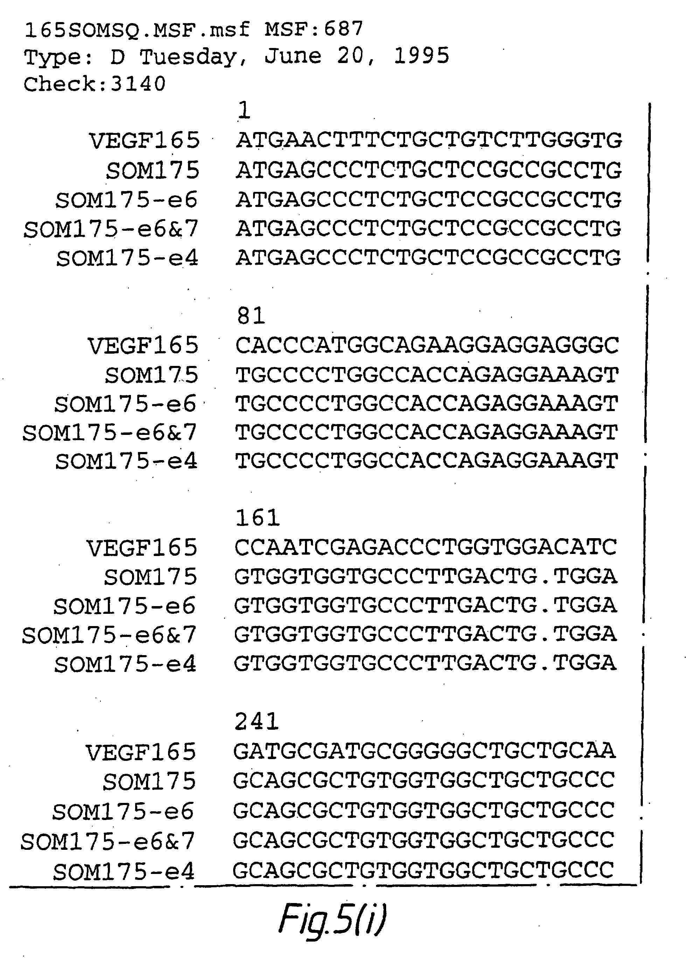

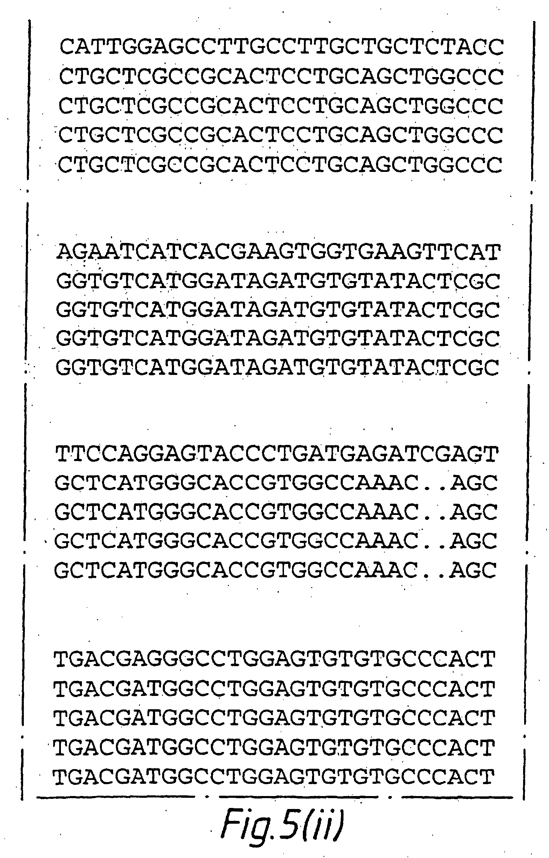

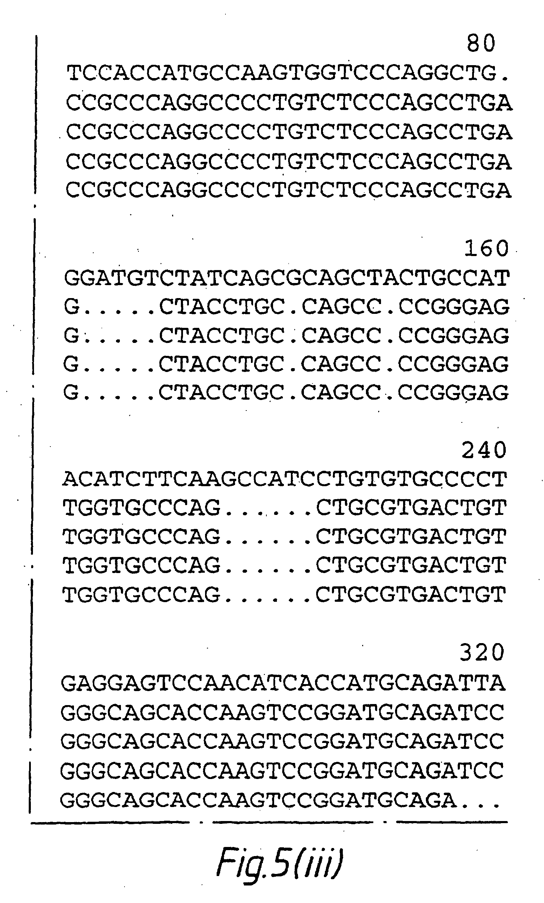

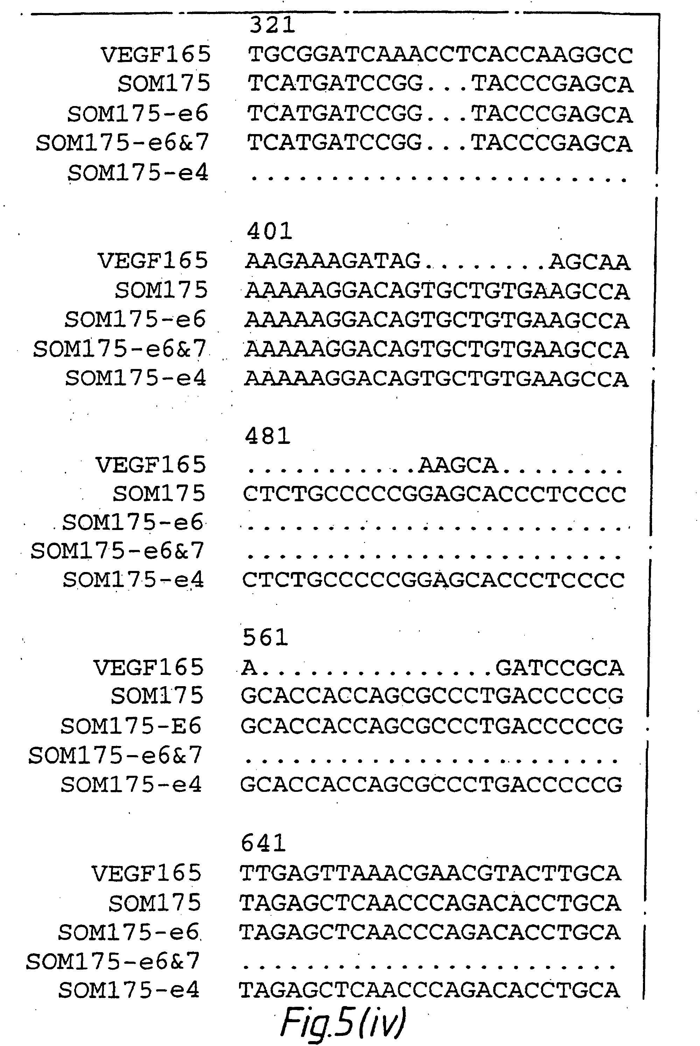

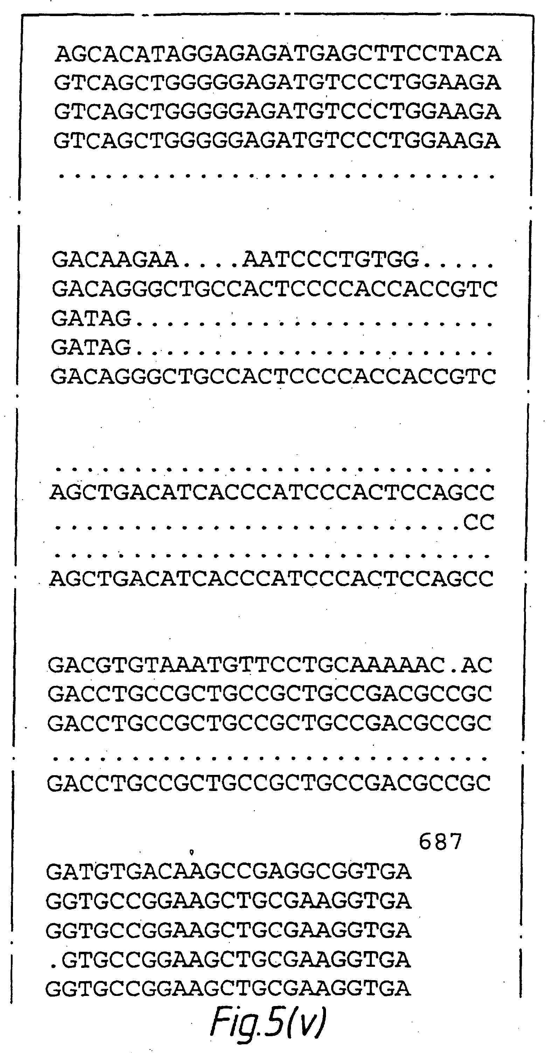

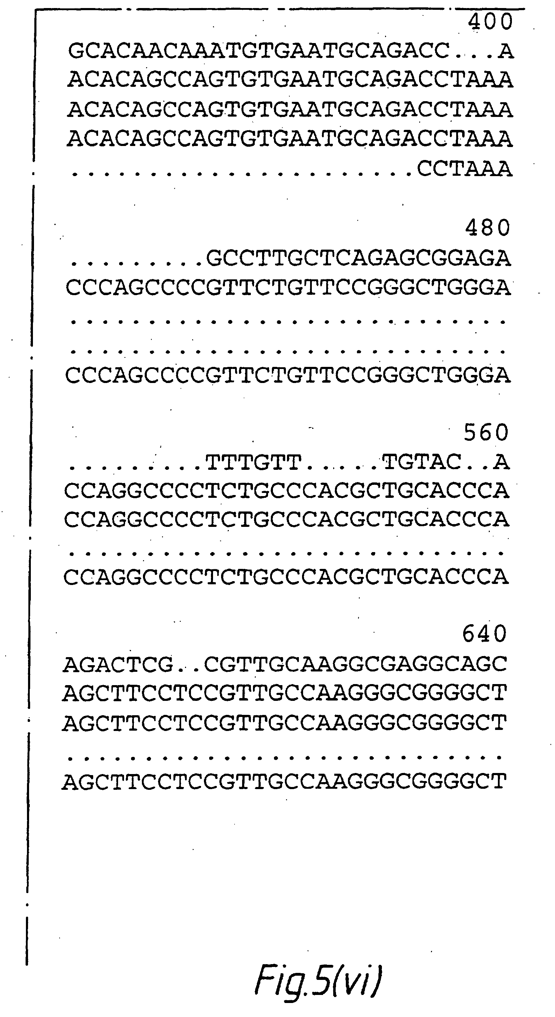

Database homology searches were performed using the BLAST algorithm (run at NCBI, USA). This analysis revealed homology to several mammalian forms of VEGF (see Figure 3). The amount of homology between SOM175 and human VEGF₁₆₅ was determined using the BESTFIT program (GCG, University of Wisconsin; see Figures 4 and 5). Nucleotide homology was estimated at 69.7% and protein homology was estimated as at least 33.3% identity and 52.5% conservation using BESTFIT analysis. BLAST analysis on nucleotide sequences revealed the almost complete match to a human expressed sequence tag EST06302 (Adams *et al*., 1993).

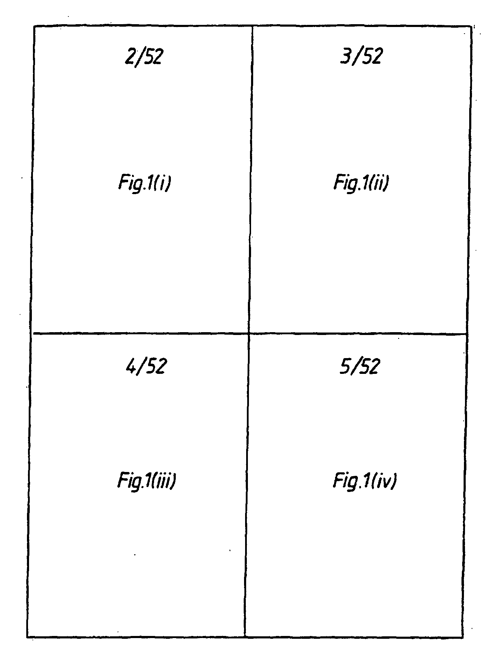

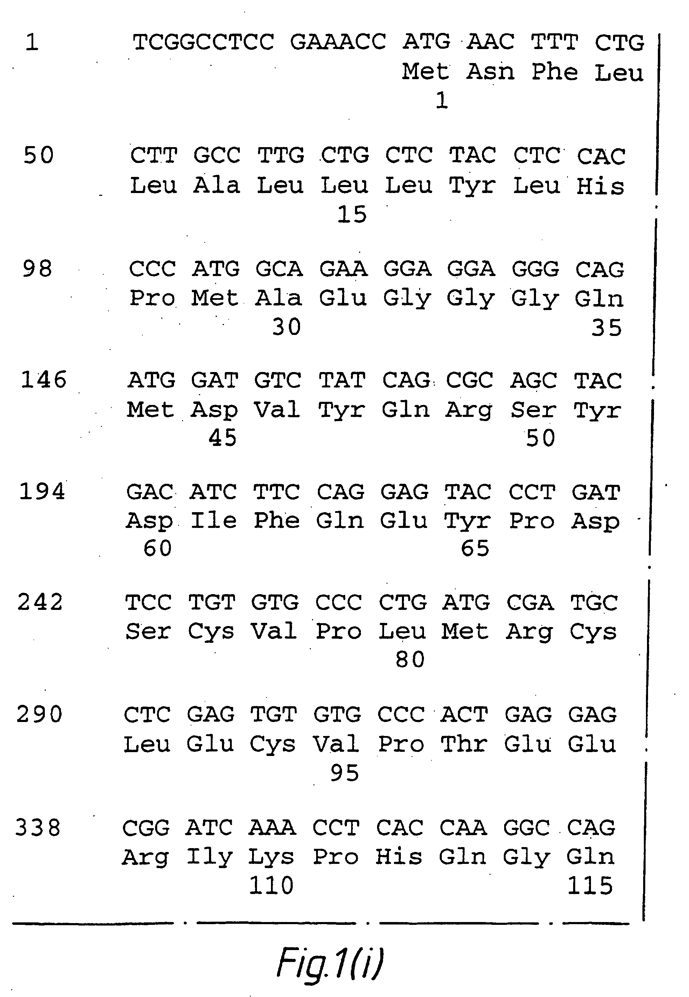

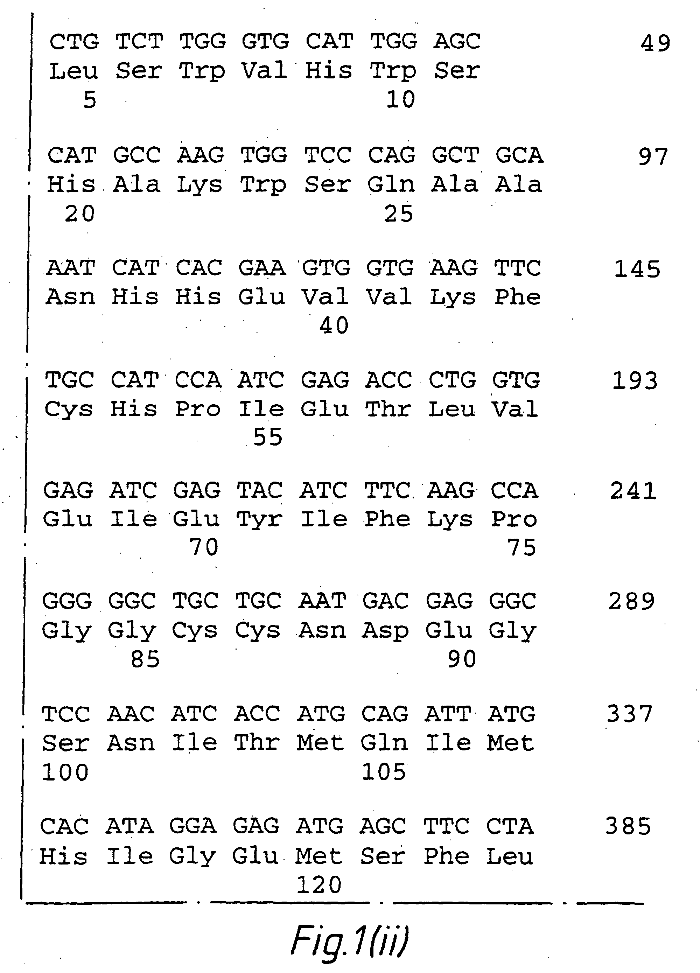

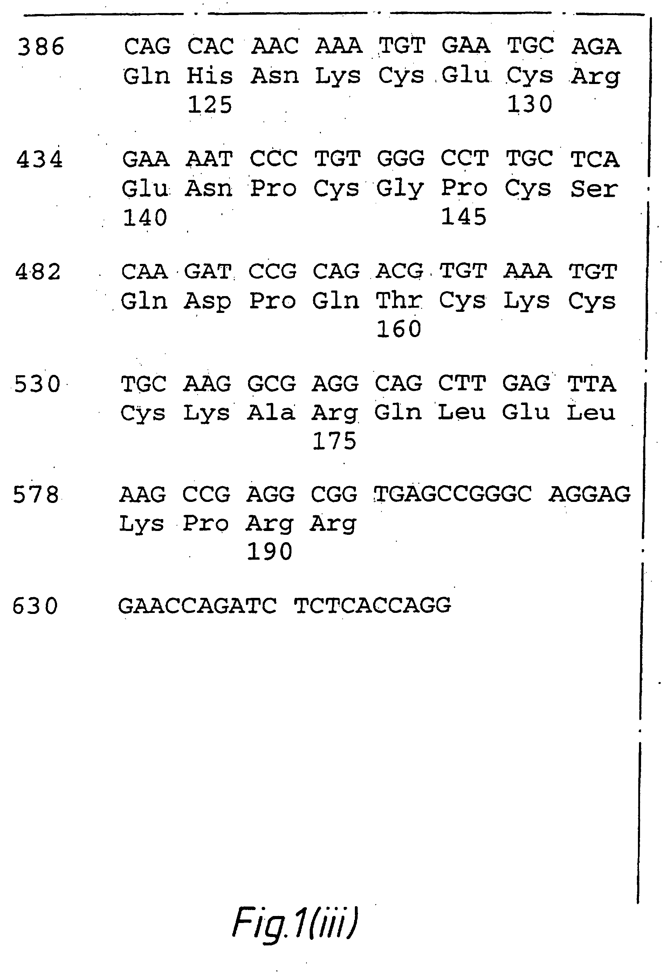

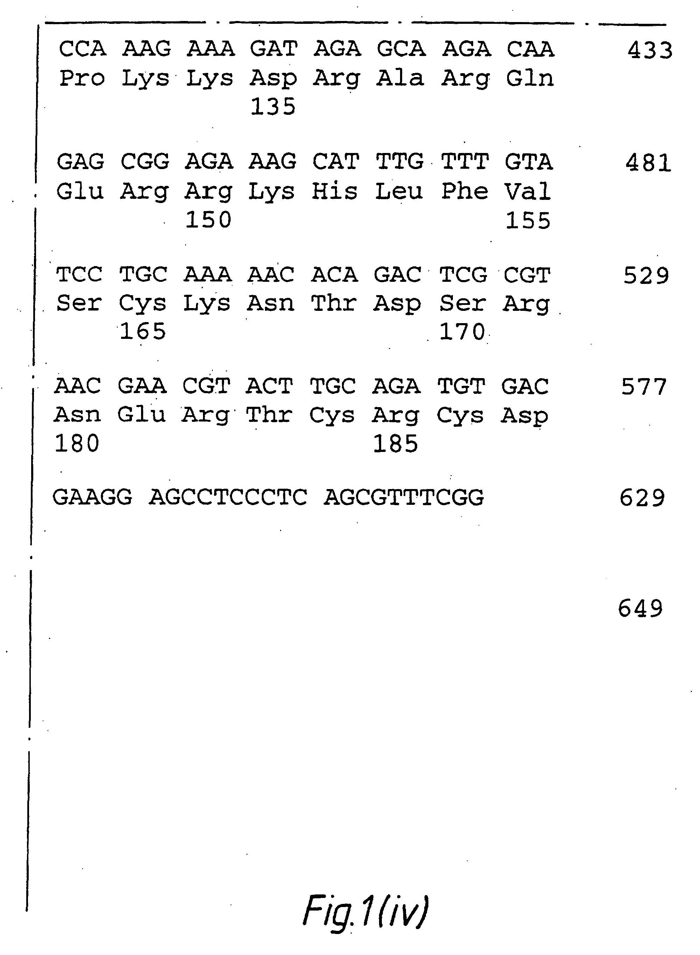

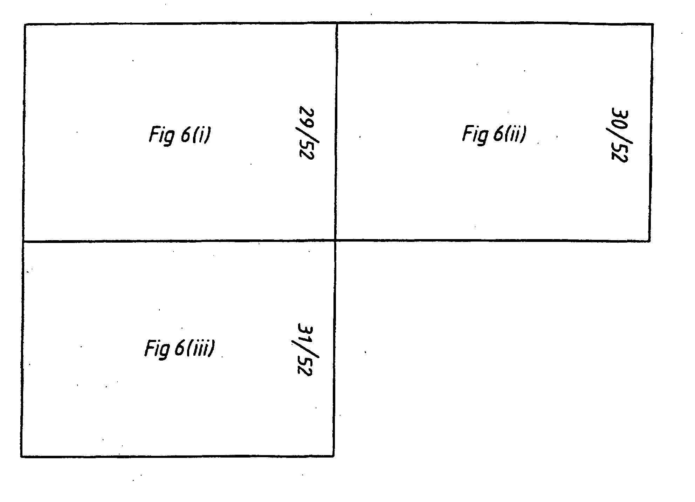

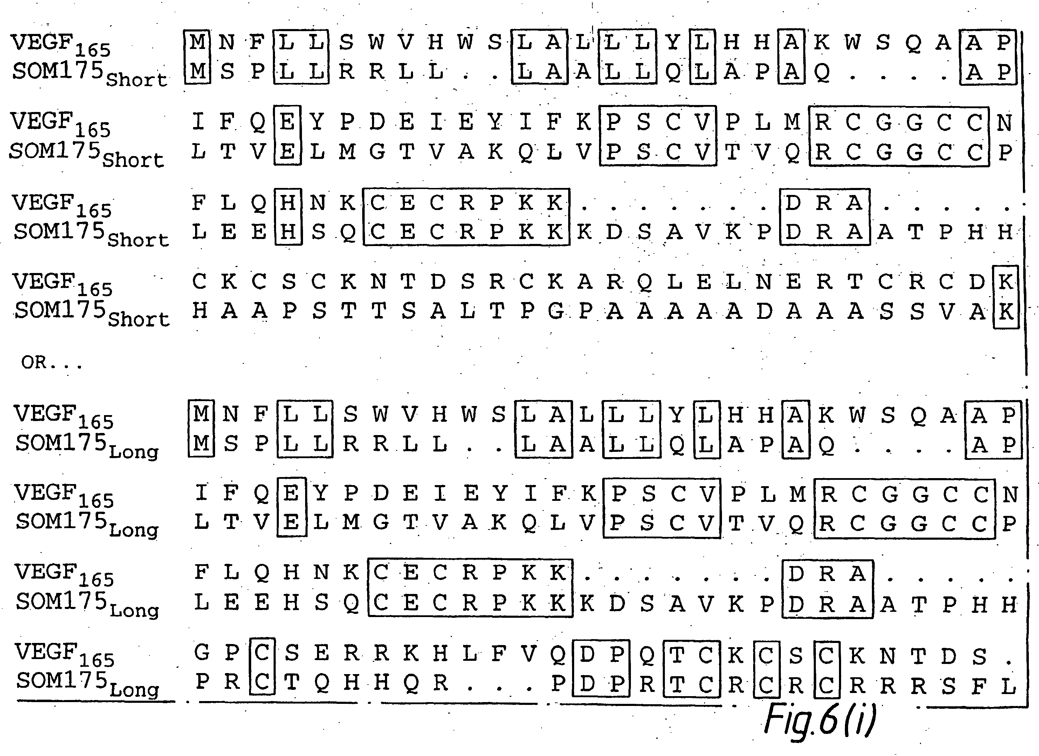

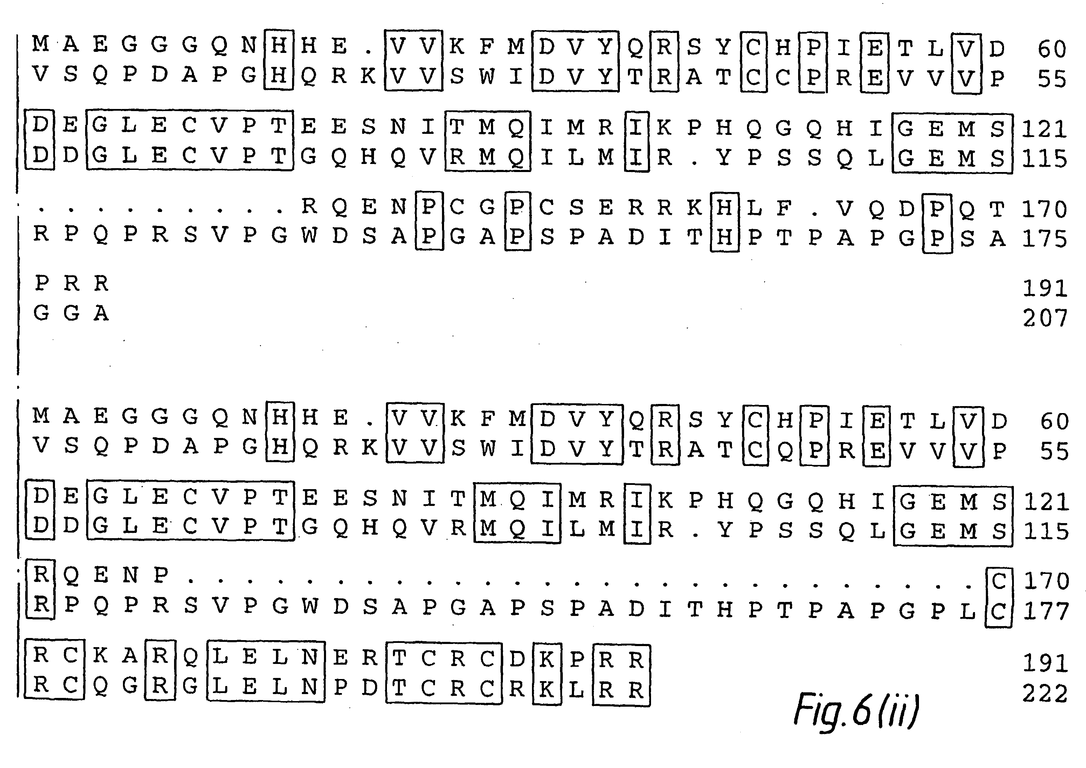

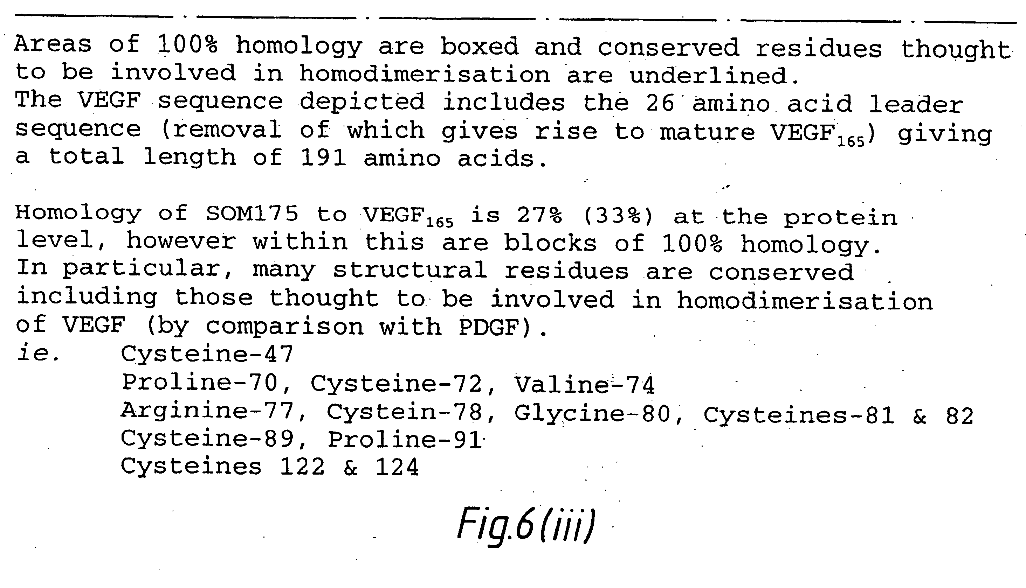

These data indicate that SOM175 encodes a growth factor that has structural similarities to VEGF. Both genes show start and stop codons in similar positions and share discrete blocks of homology. All 8 cysteines as well as a number of other VEGF residues believed to be involved in dimerisation are conserved. These residues are Cysteine-47, Proline-70, Cysteine-72, Valine-74, Arginine-77, Cysteine-78, Glycine-80, Cysteine-81, Cysteine-82, Cysteine-89, Proline-91, Cysteine-122 and Cysteine-124 and are shown in Figure 6. Given the structural conservation between VEGF and the SOM175 gene product it is also possible that they share functional similarities. It is proposed that SOM175 encodes a VEGF-like molecule that shares some properties with VEGF but has unique properties of its own. The nucleotide sequence and corresponding amino acid sequence of VEGF₁₆₅ is shown in Figure 1.

### EXAMPLE 3

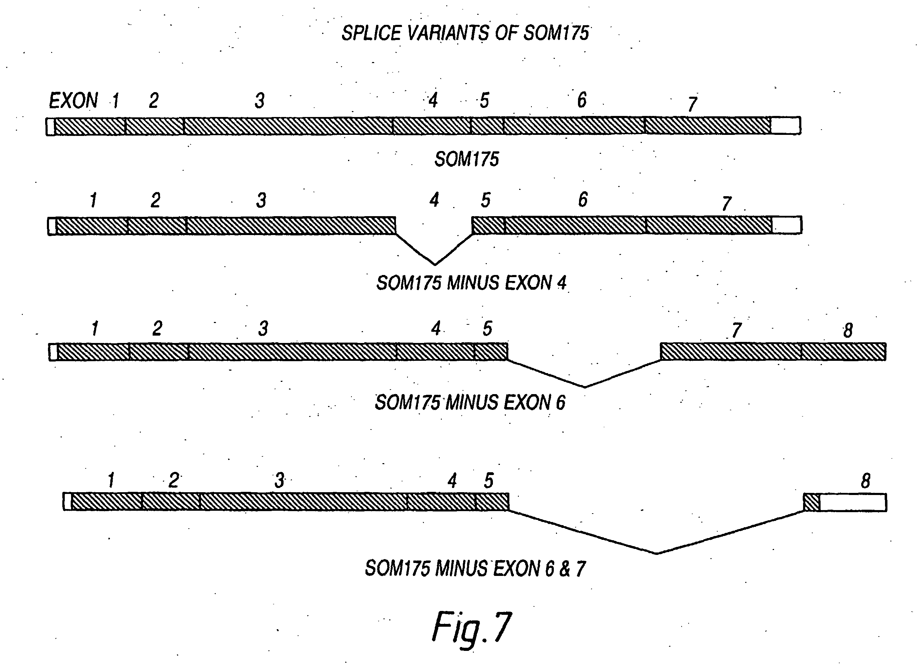

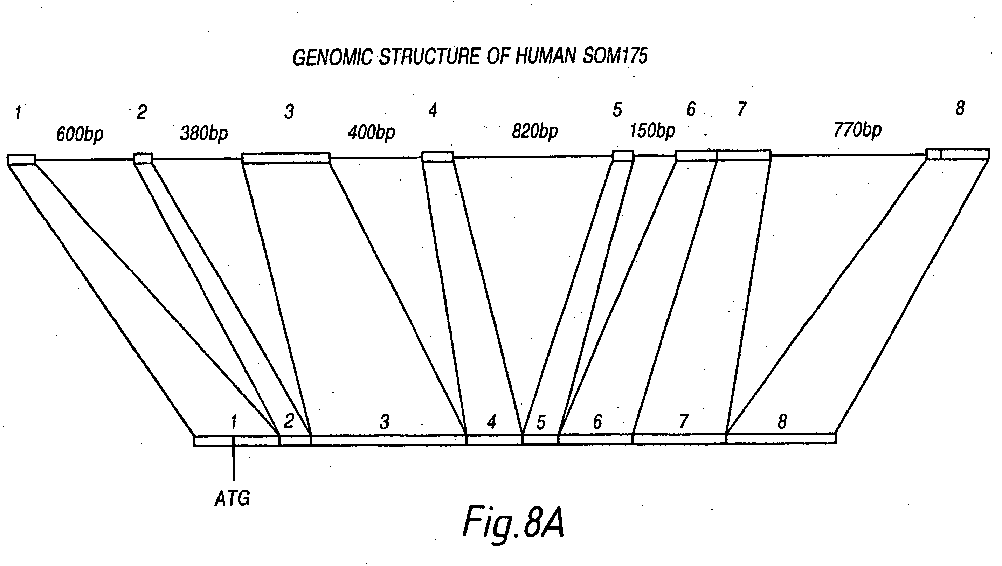

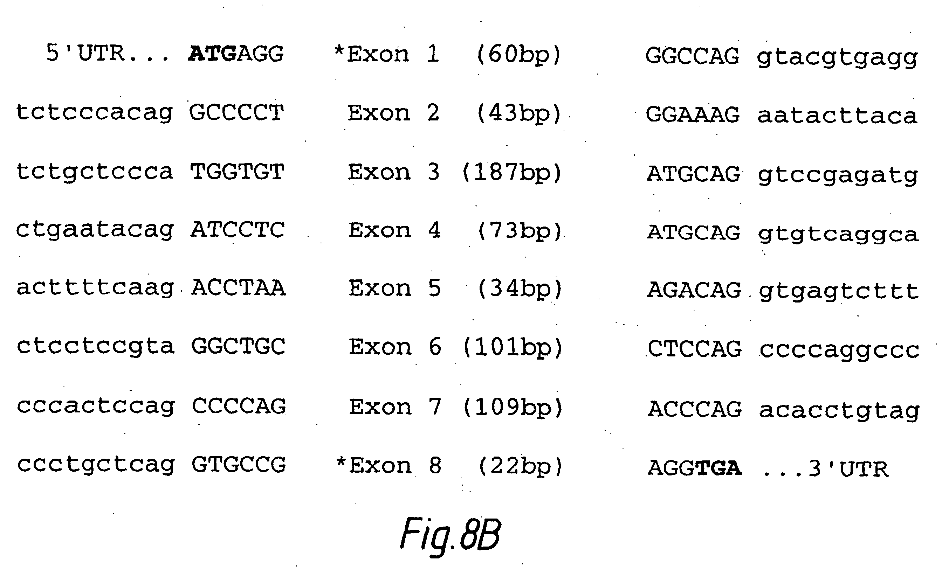

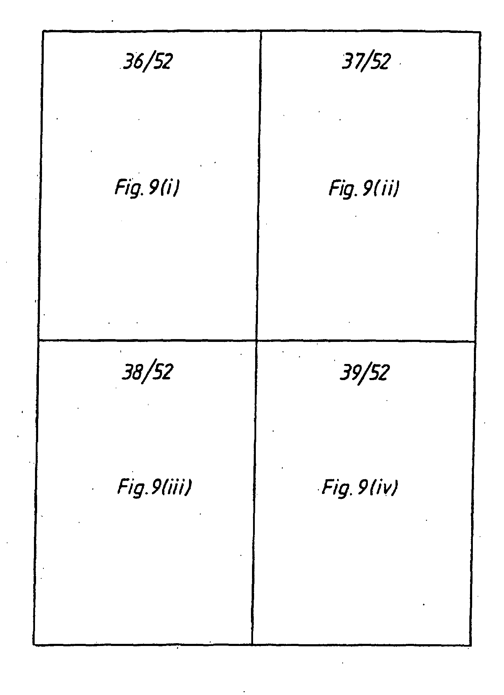

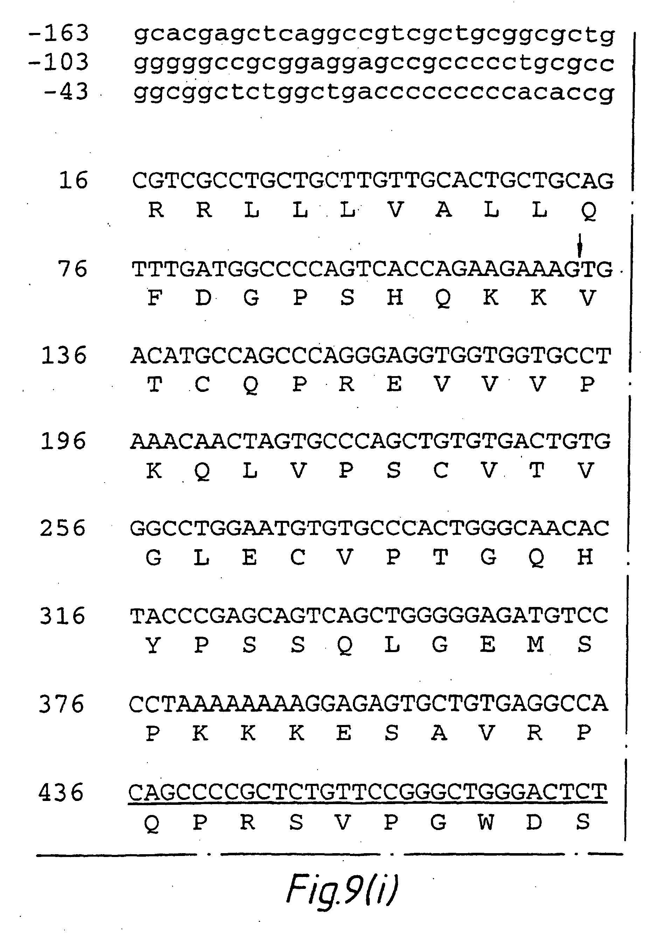

The percentage similarity and divergence between VEGF₁₆₅ family and SOM175 family (protein) were analysed using the Clustal method, MegAlign Software, DNASTAR, Wisconsin. The results are shown in Tables 2.1 and 2.2. The alternatively spliced forms of SOM175 are abbreviated to SOM715-e6 where all of exon 6 is deleted; SOM715-e6 and 7 where all of exons 6 and 7 are deleted; and SOM175-e4 where all of exon 4 is deleted. The spliced form of SOM175 are shown in Figure 7. Genomic maps of SOM175 showing intron/exon boundaries are shown in Figure 8a and 8b.

**Table 2.1**

| **A Percent nucleotide similarity between splice variants of SOM175 and human VEGF**_{**165**} | | | | | |
|---|---|---|---|---|---|
| | **VEGF**_{**165**} | **SOM175** | **SOM175-e6** | **SOM175-e6&7** | **SOM175-e4** |
| **VEGF**_{**165**} | *** | 34.9 | 39.7 | 41.4 | 37.0 |
| **SOM175** | | *** | 98.9 | 95.1 | 99.2 |
| **SOM175-e6** | | | *** | 98.8 | 84.0 |
| **SOM175-e6&7** | | | | *** | 80.3 |
| **SOM175-e4** | | | | | *** |

| **B Percent nucleotide divergence between splice variants of SOM175 and human VEGF**_{**165**} | | | | | |
|---|---|---|---|---|---|
| | **VEGF**_{**165**} | **SOM175** | **SOM175-e6** | **SOM175-e6&7** | **SOM175-e4** |
| **VEGF**_{**165**} | *** | 41.7 | 41.6 | 41.7 | 41.8 |
| **SOM175** | | *** | 0.2 | 0.2 | 0.0 |
| **SOM175-e6** | | | *** | 0.0 | 0.2 |
| **SOM175-e6&7** | | | | *** | 0.3 |
| **SOM175-e4** | | | | | *** |

**Table 2.2**

| **A Percent amino acid identity between splice variants of SOM175 and human VEGF**_{**165**} | | | | | |
|---|---|---|---|---|---|
| | **VEGF**_{**165**} | **SOM175** | **SOM175-e6** | **SOM175-e6&7** | **SOM175-e4** |
| **VEGF**_{**165**} | *** | 31.4 | 42.3 | 33.5 | 40.6 |
| **SOM175** | | *** | 74.7 | 73.7 | 99.1 |
| **SOM175-e6** | | | *** | 76.8 | 99.1 |
| **SOM175-e6&7** | | | | *** | 99.1 |
| **SOM175-e4** | | | | | *** |

| **B Percent amino acid divergence between splice variants of SOM175 and human VEGF**_{**165**} | | | | | |
|---|---|---|---|---|---|
| | **VEGF**_{**165**} | **SOM175** | **SOM175-e6** | **SOM175-e6&7** | **SOM175-e4** |
| **VEGF**_{**165**} | *** | 65.7 | 55.4 | 54.6 | 57.4 |
| **SOM175** | | *** | 19.9 | 4.2 | 0.0 |
| **SOM175-e6** | | | *** | 0.0 | 0.0 |
| **SOM175-e6&7** | | | | *** | 0.0 |
| **SOM175-e4** | | | | | *** |

### EXAMPLE 4

### BIOASSAYS TO DETERMINE THE FUNCTION OF SOM175

Assays are conducted to evaluate whether SOM175 has similar activities to VEGF on endothelial cell function, angiogenesis and wound healing. Other assays are performed based on the results of receptor binding distribution studies.

### Assays of endothelial cell function

*Endothelial cell proliferation*. Endothelial cell growth assays as described in Ferrara & Henzel (1989) and in Gospodarowicz *et al* (1989).

*Vascular permeability assay.* This assay, which utilises the Miles test in guinea pigs, will be performed as described in Miles & Miles (1952).

*Cell adhesion assay*. The influence of SOM175 on adhesion of polymorphs to endothelial cells is analysed.

*Chemotaxis*. This is performed using the standard Boyden chamber chemotaxis assay.

*Plasminogen activator assay.* Endothelial cells are tested for plasminogen activator and plasminogen activator inhibitor production upon addition of SOM175 (Pepper *et al* (1991)).

*Endothelial cell migration assay.* The ability of SOM175 to stimulate endothelial cells to migrate and form tubes is assayed as described in Montesano *et al* (1986).

### Angiogenesis Assay

SOM175 induction of an angiogenic response in chick chorioallantoic membrane is evaluated as described in Leung *et al* (1989).

Possible neurotrophic actions of SOM175 are assessed using the following assays:

### Neurite outgrowth assay and gene induction (PC12 cells)

PC12 cells (a phaeochromocytoma cell line) respond to NGF and other neurotrophic factors by developing the characteristics of sympathetic neurons, including the induction of early and late genes and the extension of neurites. These cells are exposed to SOM175 and their response monitored (Drinkwater *et al* (1991); and Drinkwater *et al* (1993)).

### Cultured neurons from the Peripheral Nervous System (PNS)

Primary cultures of the following PNS neurons are exposed to SOM175 and monitored for any response:
- sensory neurons from neural crest and dorsal root ganglia
- sympathetic neurons from sympathetic chain ganglia
- placode derived sensory neurons from nodose ganglia
- motoneurons from spinal cord
The assays are described in Suter *et al* (1992) and in Marinou *et al* (1992).

Where an *in vitro* response is observed, *in vivo* assays for properties such as uptake and retrograde transport are performed as described in Hendry *et al* (1992).

### Nerve regeneration (PNS)

Where neurotrophic effects of SOM175 are observed, its possible role in the regeneration of axotomised sensory neurons, sympathetic neurons and motoneurons is analysed by the methods of Otto *et al* (1989); Yip *et al* (1984) and Hendry *et al* (1976).

### Actions of SOM175 on CNS neurons

The ability of SOM175 to promote survival of central nervous system neurons is analysed as described in Hagg *et al* (1992); Williams *et al* (1986); Hefti (1986) and Kromer (1987).

### Wound Healing

The ability of SOM175 to support wound healing are tested in the most clinically relevant model available, as described in Schilling *et al* (1959) and utilised by Hunt *et al* (1967).

### The Haemopoietic System

A variety of *in vitro* and *in vivo* assays on specific cell populations of the haemopoietic system are available and are outlined below:
*Stem Cells*
*Murine*
A variety of novel *in vitro* murine stem cell assays have been developed using FACS-purified cells:

### (a) Repopulating Stem Cells

These are cells capable of repopulating the bone marrow of lethally irradiated mice, and have the Lin⁻, Rh^{hi}, Ly-6A/E⁺, c-kit⁺ phenotype. The test substance is tested on these cells either alone, or by co-incubation with multiple factors, followed by measurement of cellular proliferation by ³H thymidine incorporation.

### (b) Late Stage Stem Cells

These are cells that have comparatively little bone marrow repopulating ability but can generate D13 CFU-S. These cells have the Lin⁻, Rh^{hi}, Ly-6A/E⁺, c-kit⁺ phenotype. The test substance is incubated with these cells for a period of time, injected into lethally irradiated recipients, and the number of D13 spleen colonies enumerated.

### (c) Progenitor-Enriched Cells

These are cells that respond *in vitro* to single growth factors, and have the Lin⁻, Rh^{hi}, Ly-6A/E⁺, c-kit⁺ phenotype. This assay will show if SOM175 can act directly on haemopoietic progenitor cells. The test substance is incubated with these cells in agar cultures, and the number of colonies enumerated after 7-14 days.

### Atherosclerosis

Smooth muscle cells play a crucial role in the development or initiation of atherosclerosis, requiring a change in their phenotype from a contractile to a synthetic state. Macrophages, endothelial cells, T lymphocytes and platelets all play a role in the development of atherosclerotic plaques by influencing the growth and phenotypic modulations of smooth muscle cell. An *in vitro* assay that measures the proliferative rate and phenotypic modulations of smooth muscle cells in a multicellular environment is used to assess the effect of SOM175 on smooth muscle cells. The system uses a modified Rose chamber in which different cell types are seeded onto opposite coverslips.

### Effects of SOM175 on bone

The ability of SOM175 to regulate proliferation of osteoblasts is assayed as described in Lowe *et al* (1991). Any effects on bone resorption are assayed as described in Lowe *et al* (1991). Effects on osteoblast migration and changes in intracellular molecules (e.g. cAMP accumulation, alkaline phosphatase levels) are analysed as described in Midy *et al* (1994).

### Effects on skeletal muscle cells

Effects of SOM175 on proliferation of myoblasts and development of myotubes can be determined as described by Ewton *et al* (1980) and by Gospodarowicz *et al* (1976).

### EXAMPLE 5

### CLONING MURINE SOM175 DNA

### Isolation of cDNAs

Murine SOM175 (mSOM175) clones were selected from a lambda Zap new born whole brain cDNA library (Stratagene). Primary phage from high density filters (5 x 10⁴ pfu/plate) were identified by hybridisation with a 682bp ³²P-labelled probe generated by PCR from an hVRF cDNA (pSOM175) as described above. Hybridisation and stringent washes of nylon membranes (Hybond-N) were carried out at 65°C under conditions described by Church and Gilbert (1984). Positive plaques were picked, purified and excised *in vivo* to produce bacterial colonies containing cDNA clones in pBluescript SK-.

### Isolation of genomic clones

Genomic clones were isolated from a mouse strain SV/129 library cloned in the lambda Fix II vector. (Stratagene). High density filters (5 x 10⁴ pfu/filter) were screened with a 563 bp ³²P-labelled probe generated by PCR amplification of the nucleotide 233-798 region of the mSOM175 cDNA (see Figure 9). Positive clones were plugged and re-screened with filters containing 400-800 pfu. Large scale phage preparations were prepared using the QIAGEN lambda kit or by ZnCl₂ purification (Santos, 1991).

### Nucleotide sequencing and analysis

cDNAs were sequenced on both strands using a variety of vector-based and internal primers with Applied Biosystems Incorporated (ABI) dye terminator sequencing kits according to the manufacturer's specifications. Sequences were analysed on an ABI Model 373A automated DNA sequencer. Peptide homology alignments were performed using the program BESTFTT (GCG, Wisconsin).

### Identification of intron/exon boundaries

Identification of exon boundaries and flanking regions was carried out using PCR with mouse genomic DNA or mSOM175 genomic lambda clones as templates. The primers used in PCR to identify introns were derived from the hSOM175 sequence and to allow for potential human-mouse sequence mismatches annealing temperatures 5-10°C below the estimated Tₘ were used. All PCR products were sized by agarose gel electrophoresis and gel purified using QIAquick spin columns (Qiagen) and the intron/exon boundaries were. sequenced directly from these products. In addition, some splice junctions were sequenced from subcloned genomic fragments of mSOM175. Intron/exon boundaries were identified by comparing. cDNA and genomic DNA sequences.

### Northern analysis

Total cellular RNA was prepared from a panel of fresh normal adult mouse tisues (brain, kidney, liver, muscle) using the method of Chomczynski and Sacchi (1987). 20µg of total RNA were electrophoresed, transferred to a nylon membrane (Hybond N, Amersham) and hybridised under standard conditions (Church & Gilbert, 1984). Filters were washed at 65°C in 0.1xSSC (20xSSC is 3M NaCl/0.3M trisodium citrate), 0.1% SDS and exposed to X-ray film with intensifying screens at -70°C for 1-3 days.

### Characterisation of mSOM175 cDNAs

Murine SOM175 homologues were isolated by screening a murine cDNA library with an hSOM175 cDNA clone. Five clones of sizes varying from 0.8-1.5 kb were recovered and sequenced. The cDNA sequences were complied to give a full length 1041 bp cDNA sequence covering the entire open reading frame (621 bp or 564 bp depending on the splice form, see below) and 3' UTR (379 bp), as well as 163 bp of the 5' UTR (Figure 9).

The predicted initiation codon matched the position of the start codon in hSOM175. One other out of frame ATG was located at position -47 and two termination codons were observed upstream (positions -9 and -33, respectively) and in-frame with the putative initiation codon.

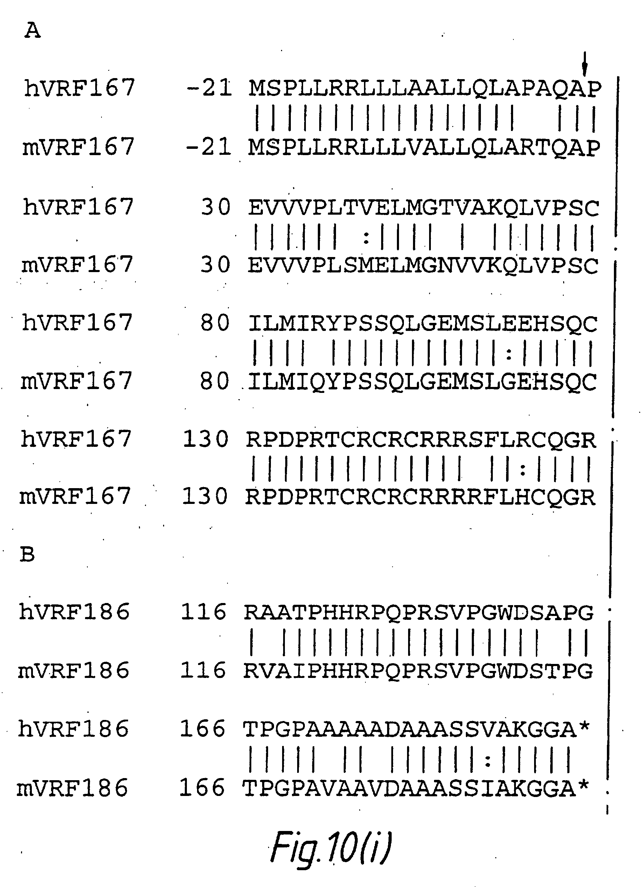

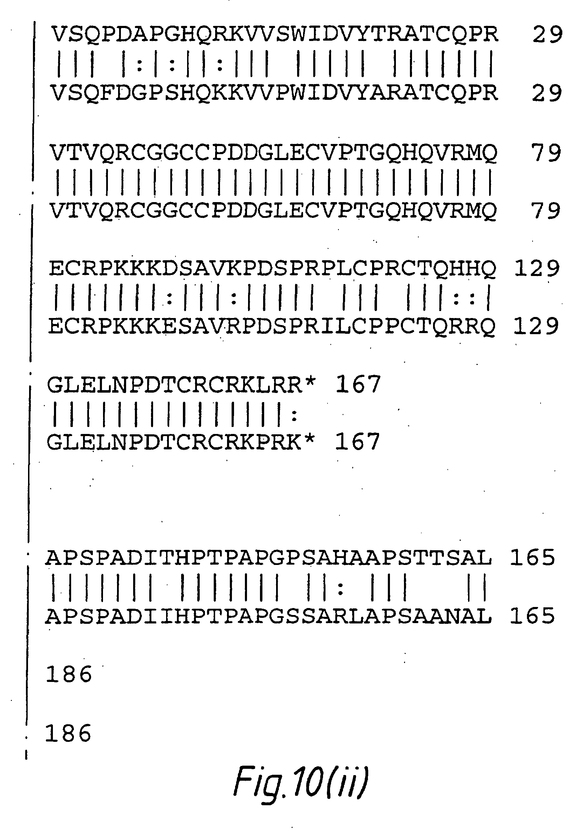

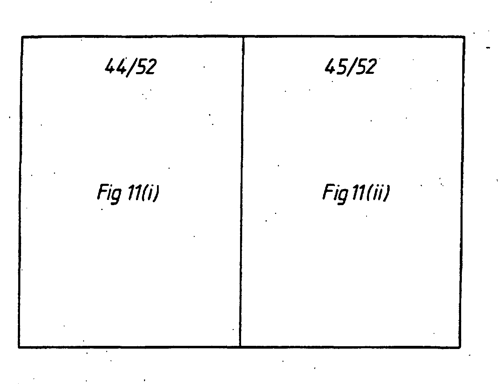

The predicted N-terminal signal peptide of hSOM175 appears to be present in mSOM175 with 81% identity (17/21 amino acids). Peptide cleavage within mSOM175 is expected to occur after residue 21 (Figure 10). These data suggest that mature mSOM175 is secreted and could therefore conceivably function as growth factor.

As with hSOM175, two open reading frames (ORFs) were detected in cDNAs isolated by library screening. Four of five clones were found to be alternatively spliced and lacked a 101 bp fragment homologous to exon 6 of hSOM175. The predicted peptide sequences of the two isoforms of mSOM175 were determined and aligned with the corresponding human isoforms (Figure 10).

The message encoding mSOM175₁₈₆ contains a 621 bp ORF with coding sequences terminating at position +622, towards the end of exon 7 (Figure 9). The smaller message encoding mSOM175₁₆₇ actually terminates downstream of the +622 TAG site due to a frame shift resulting from splicing out of the 101 bp exon 6 and the introduction of a stop codon (TGA) at position +666, near the beginning of exon 8 (Figure 9).

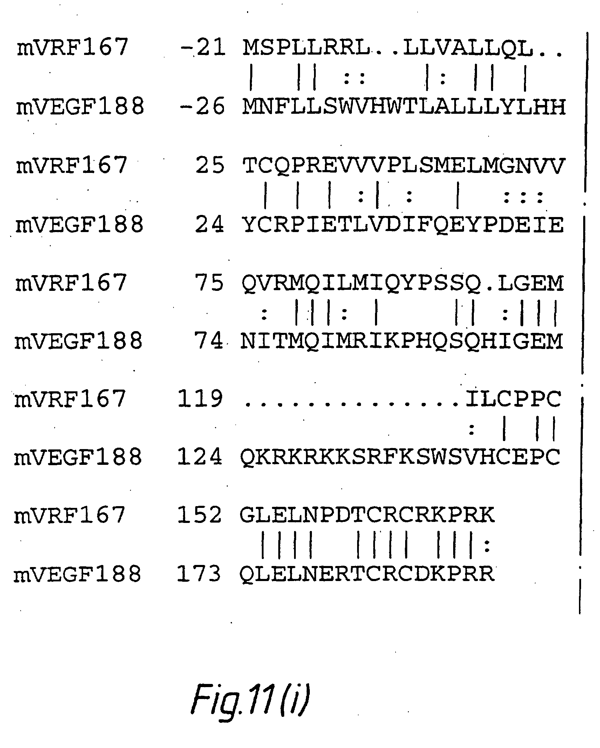

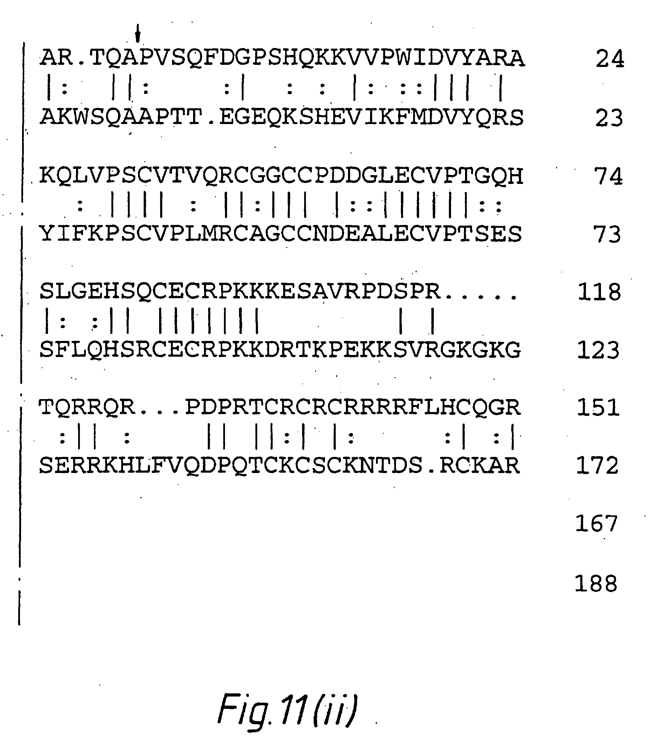

The mSOM175₁₈₆ protein has strong homology to the amino and central portions of VEGF while the carboxyl end is completely divergent and is alanine rich. mSON175₁₆₇ possesses these similarities and also maintains homology to mVEGF right through to the C-terminus (Figure 11). The overall homology of mSOM175₁₆₇ to hSOM175₁₆₇ was 85% identity and 92% similarity, respectively (Figure 10). Likewise, homology between mSOM175₁₆₇ and mVEGF (Breier *et al*, 1992) was 49% identity and 71% conservative amino acid substitution, respectively (Figure 11).

A canonical vertebrate polyadenylation signal (AATAAA) (Birnstiel *et al*, 1986) was not present in the mSOM175 cDNA, however, the closely matching sequence GATAAA is present at similar positions in both mouse and human SOM175 cDNAs (Figure 9). In contrast to hSOM175, mSOM175 was found to contain an AC dinucleotide repeat at the extreme 3' end of the 3' UTR (nucleotide positions 998 to 1011, Figure 9). Polymorphism of this repeat region was observed between some of the mSOM175 cDNAs, with the number of dinucleotides varying from 7 to 11.

### Genomic Characterisation of mSOM175

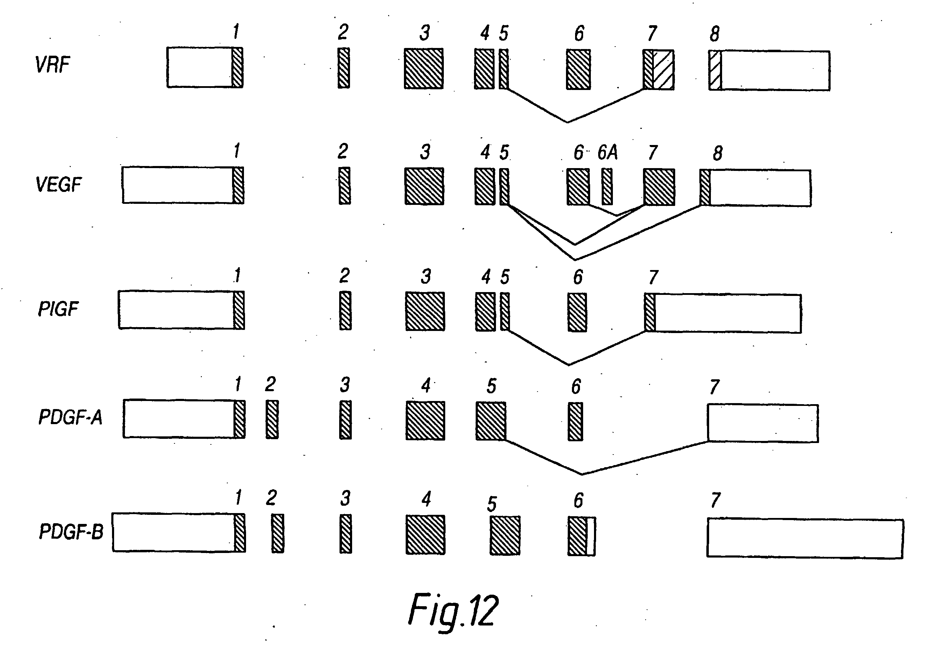

Intron/exon boundaries (Table 3) were mapped using primers which flanked sequences homologous to the corresponding hSOM175 boundaries. Introns I, III, IV and VI of mSOM175 (Table 3, Figure 12) were smaller than the hSOM175 intervening sequences. The complete genomic sequence was compiled from the 5' UTR of mSOM175 through to intron VI, the largest intervening region (2.2 kb), by sequencing amplified introns and cloned genomic portions of mSOM175. There was only one major difference in genomic structure between mSOM175 and hSOM175 and that was the exon 7/intron VI boundary of mSOM175 was located 10bp further downstream in relation to the cDNA sequence, hence exon 7 in mSOM175 is 10bp longer; than the corresponding exon in hSOM175.

Exons 6 and 7 are contiguous in mSOM175, as has been found to occur in the human homologue. The strong sequence homology between exon 6 of mSOM175 and hSOM175 (Figure 10) suggests that this sequence is not a retained intronic sequence but rather encodes a functional part of the SOM175₁₈₆ isoform.

General intron/exon structure is conserved between the various members of the VEGF gene family (VEGF, PIGF, hSOM175) and therefore it is not surprising that the overall genomic organisation of the mSOM175 gene is very similar to these genes (Figure 12).

Previous comparative mapping studies have shown that the region surrounding the human multiple endocrine neoplasia type 1 disease locus on chromosome 11q13 is syntenic with the proximal segment of mouse chromosome 19 (Rochelle *et al*, 1992). Since the inventors have mapped the hSOM175 gene to within 1kb of the human *MEN1* locus (see above) it is most likely that the murine SOM175 gene maps near the centromere of chromosome 19.

### Expression studies of mSOM175

Northern analysis of RNA from adult mouse tissues (muscle, heart, lung and liver) showed that expression appears to be ubiquitous and occurs primarily as a major band of approximately 1.3kb in size (Figure 14). This is somewhat different to the pattern observed for hSOM175 in which two major bands of 2.0 and 5.5 kb have been identified in all tissues examined. The 1.3 kb murine message presumably corresponds to the shorter of the human transcripts and the size variation thereof is most likely due to a difference in the length of the respective 5' UTRs.

### EXAMPLE 6

### EXPRESSION OF MURINE SOM175 IN PRE- AND POST-NATAL MOUSE

### Animals

Timed pregnant (n=4) and young adult (n=2) mice (C57 inbred strain, ALAB, Sweden) were sacrificed with carbon dioxide, and the relevant tissues were taken out and frozen on a chuck. Tissues were kept at -70°C until further use. Two gestational ages was used in this study; embryonic day 8 (E8), 14 and E17.

### In situ hybridisation histochemistry

*In situ* hybridisation was performed as previously described (Dagerlind *et al*, 1992). Briefly, transverse sections (14µm) were cut in a cryostat (Microm, Germany), thawed onto Probe-On slides (Fisher Scientific, USA) and stored in black sealed boxes at -70°C until used. The sequences of the synthetic 42-mer oligonucleotides complementary to mRNA encoding mSOM175 were
ACCACCACCTCCCTGGGCTGGCATGTGGCACGTGCATAAACG [SEQ ID NO:11] (complementary to nt 120-161) and
AGTTGTTTGACCACATTGCCCATGAGTTCCATGCTCAGAGGC [SEQ ID NO:12] (complementary to nt 162-203). To detect the two alternative splice forms oligonucleotide GATCCTGGGGCTGGAGTGGGATGGATGATGTCAGCTGG [SEQ ID NO:13] (complementary to nt xxx-xxx) and
GCGGGCAGAGGATCCTGGGGCTGTCTGGCCTCACAGCACT [SEQ ID NO:14] were used. The probes were labeled at the 3'-end with deoxyadenosine-alpha[thio]triphosphate [³⁵S] (NEN, USA) using terminal deoxynucleotidyl transferase (IBI, USA) to a specific activity of 7-10 x 10⁸ cpm/µg and hybridised to the sections without pretreatment for 16-18 h at 42°C. The hybridisation mixture contained: 50% v/v formamide, 4 x SSC (1 x SSC = 0.15M NaCl and 0.015M sodium-citrate), 1 x Denhardt's solution (0.02% each of polyvinyl-pyrrolidone, BSA and Ficoll). 1% v/v sarcosyl (N-lauroylsarcosine; Sigma), 0.02M phosphate buffer (pH 7.0), 10% w/v dextran sulfate (Pharmacia, Sweden), 250µg/ml yeast tRNA (Sigma), 500µg/ml sheared and heat denatured salmon sperm DNA (Sigma) and 200mM dithiothreitol (DTT; LKB, Sweden). In control sections, the specificity of both probes was checked by adding a 20-fold excess of unlabeled probe to the hybridisation mixture: In addition, adjacent sections were hybridised with a probe unrelated to this study which gave a different expression pattern. Following hybridisation the sections were washed several times in 1 x SSC at 55°C, dehydrated in ethanol and dipped in NTB2 nuclear track emulsion (Kodak, USA). After 3-5 weeks the sections were development in D-19 developer (Kodak, USA) and cover-slipped. In some cases, sections were opposed to an autoradiographic film (Beta-max autoradiography film Amersham Ltd, UK) prior to emulsion-dipping.

The four different probes gave identical hybridisation patterns in all tissues examined. Mouse SOM175 expression was detecting already in the E8 embryo, in which positive signal was recorded over structures most likely corresponding to the neuronal tube. In sagittal sections of E14 mouse embryo the strongest hybridisation signal was present over heart and in the nervous system, especially cerebral cortex (Figure 14A). A low level of expression was present in all other tissues. At a later gestational age, E17, a high mSOM175 mRNA signal was confined to he heart and brown fat tissue in the back and around the neck (Figure 14B). Clearly positive hybridisation signals were present in the gray of the spinal cord and in the tongue (Figure 14B). Expression in the cerebral cortex was clearly reduced compared to day 14. The weak background expression seen in the E14 embryo in for example muscle, had decreased at this gestational age. A strong mSOM175 mRNA hybridisation signal was present solely over the heart and in the brown fat in the young adult mice (Figure 14C). The signal over the heart was evenly distributed ove the entire ventricular wall, including the papillary muscles (Figure 14D). In sections of heart tissue hybridised with an excess of cold probe, no specific labeling over background signal was recorded (Figure 14E).

Apart from the heart, mSOM175 mRNA signal was present over certain tissues on the outside of the thoracic cage that morphologically resembled brown fat. This was verified with sudan black counterstaining, which showed a strong staining in the same areas (Figure 15A and 15B). In transverse sections of adult mouse spinal cord, the mVRF probes gave a neuronal staining pattern over the gray matter (Figure 15C). Counterstaining with toluidine (Figure 15D) showed that motoneurons in the ventral horn (Figure 15C and 15D), interneurons (Figure 15C) in the deep part of the dorsal horn and around the central canal where to a large extent positive for mSOM175:mRNA.

### EXAMPLE 7

### EFFECTS OF VEGF AND SOM175 PROTEINS ON CHICK SENSORY NEURONS

The effects of VEGF and SOM175 proteins on embryonic day 8 chick sensory neurons were determined using the method of Nurcombe *et al* (1992). The neuronal assay was read at 48 hours using 2000 cells per assay well. The results were obtained using ³H-thymidine counts. The percentage survival of neurons, neurite outgrowth and average neurite length in µm were determined using NGF as positive control and various concentrations of VEGF, VEGF in the presence of heparin and VEGF in the presence of heparin and 5 µM, 5'-flurouracil (5FU). 5FU kills glial cells.

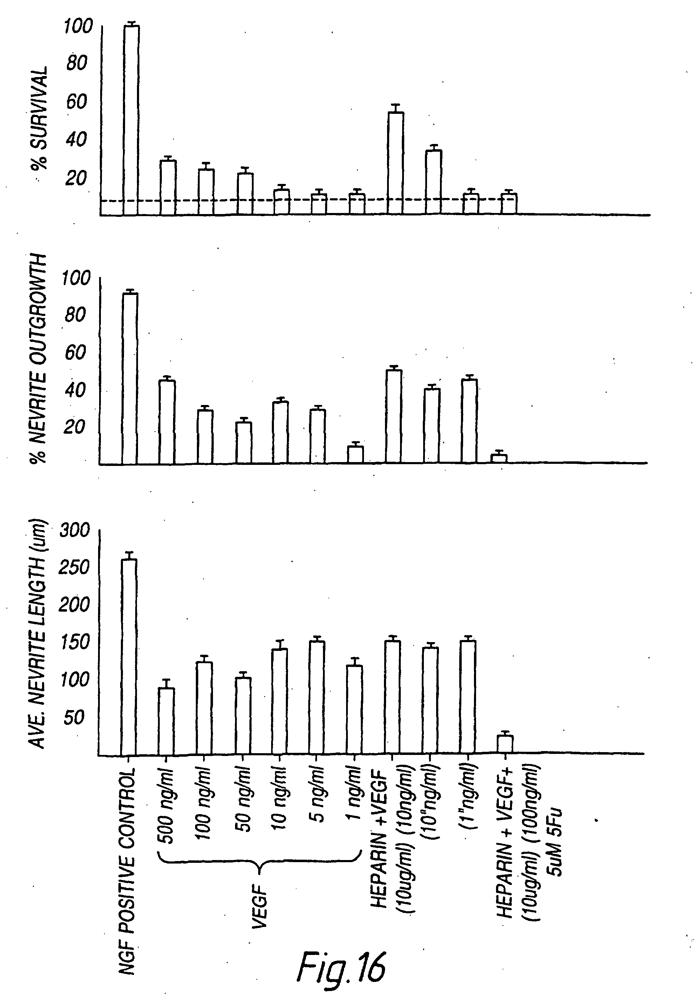

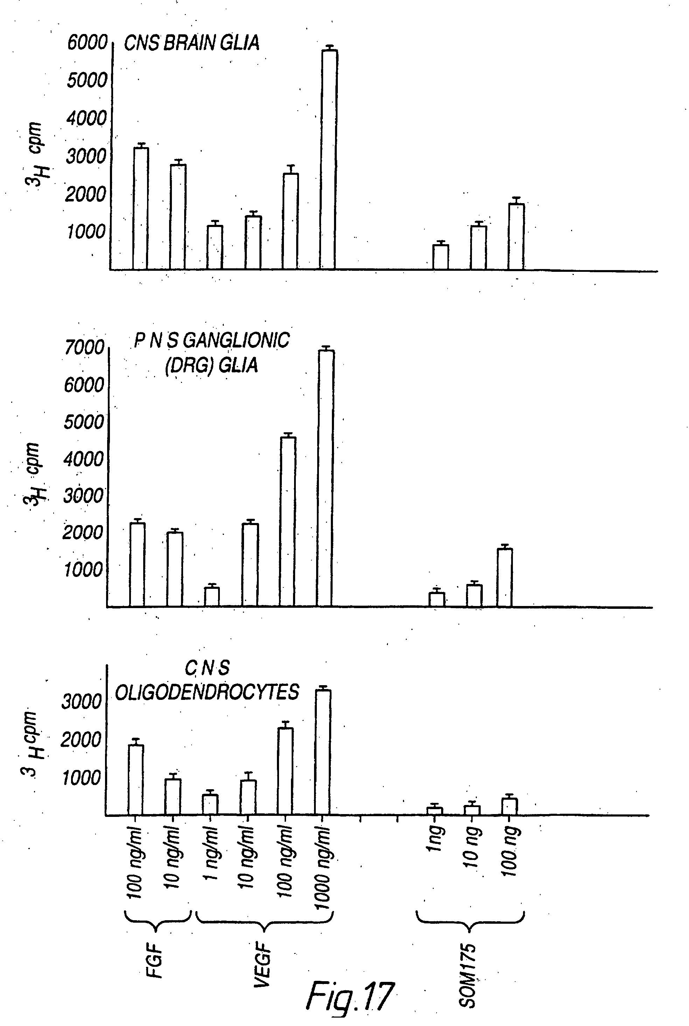

The results are shown in Figure 16. The results show that VEGF is effective in promoting neuronal survival but that this requires the presence of glial cells. Figure 17 shows the results of the effect of VEGF and SOM175 on three types of chick glia. The glia tested were CNS glia, peripheral glia and CNS oligodendrocytes. Heparin was used as 10 µg/ml in all cultures and the assay was read at 24 hours. Results were measured in ³H-thymidine counts using 2000 cells per well.

The results show that for chick central and peripheral neurons, astroglia were markedly stimulated to proliferate by SOM175 in the presence of heparin but that chick oligodendrocytes showed negligible increase in the rate of division.

### EXAMPLE 8

### EFFECTS OF SOM175 PROTEINS ON MOUSE PRIMARY AND CENTRAL NEURONS

The results in Example 7 show that VEGF isoform had an effect on chick primary and central neurons through the agency of the astroglial cells. Similar experiments were repeated in mouse cells.

### Culture conditions

Neuronal and gligal cells for all *in vitro* experiments were prepared and cultured according o the techniques described in "Methods in Neurosciences (Vol. 2): Cell Culture" Ed. P.M. Conn, Academic Press, San Diego, 1990, pp33-46 for astroglial cells, pp56-74 for oligodendroglial cells, and pp87-102 for central neurons.

Cells were plated onto 24-well culture clusters (Nunc) coated with poly-L-ornithine (0.1 mg/ml, 1h) at a density of 2,000 cells/well. After 48 hours in culture, neurons were counted in the wells under inverted phase light using well established techniques (Maruta *et al*. 1993) and glial cells assessed with [³H]thymidine uptake to monitor cell division rates as below. Heparin (10µg/ml, low molecular weight fraction, Sigma Chemical Corp.) was present at all times in the culture media except where noted. The neuronal cultures were supplemented with 5mM 5-fluoro-2-deoxyuridine (Sigma) to suppress background glial growth.

### ³H-Thymidine incorporation assay for glial cell proliferation

The cells were pulsed for 14h with ³H-thymidine (specific activity 103 µCi/ug) fraom a stock concentration of 0.1 mCi/ml in standard medium, giving a final incubating volume of 20 µl/well. The contents of the wells were harvested and absorbed onto nitrocellulose paper (Titertek, Flow). Remaining adherent cells were removed by incubation with trypsin/versene (CSL Limited, Victoria, Australia) for 5 min. This procedure was carried out twice. The nitrocellulose discs were washed in a standard Titertek harvester (Flow) using first distilled water, and then methanol. The nitrocellulose discs were dried, scintillation fluid (containing 5% v/v Triton-X) added and the discs counted on a scintillation counter.

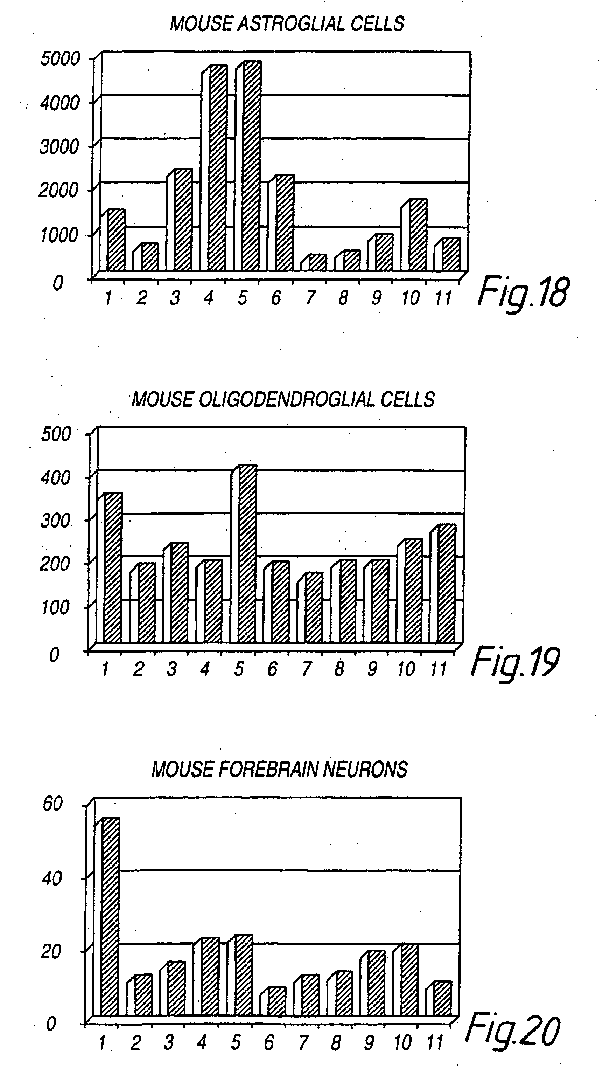

Greatest activity was seen with preparations of SOM175 absent exon 6 (SOMΔX6) on mouse astroglial cell cultures, where there was a significant stimulus to their proliferation when delivered in conjunction with heparin (Figure 16). Little stimulus was given to the proliferation of oligodendroglial cells (Figure 17), and very little discernable potentiation of the survival response of isolated forebrain neurons (Figure 18). The standard deviation on all three graphs for each point was less than 8%.

The viability of neurons can be maintained by promoting glial cell proliferation. Furthermore, SOMΔX6 is a good inducer of astroglial proliferation and may be expressed in conjunction with the formation of astroglial endfeet on central nervous system endothelial cells.

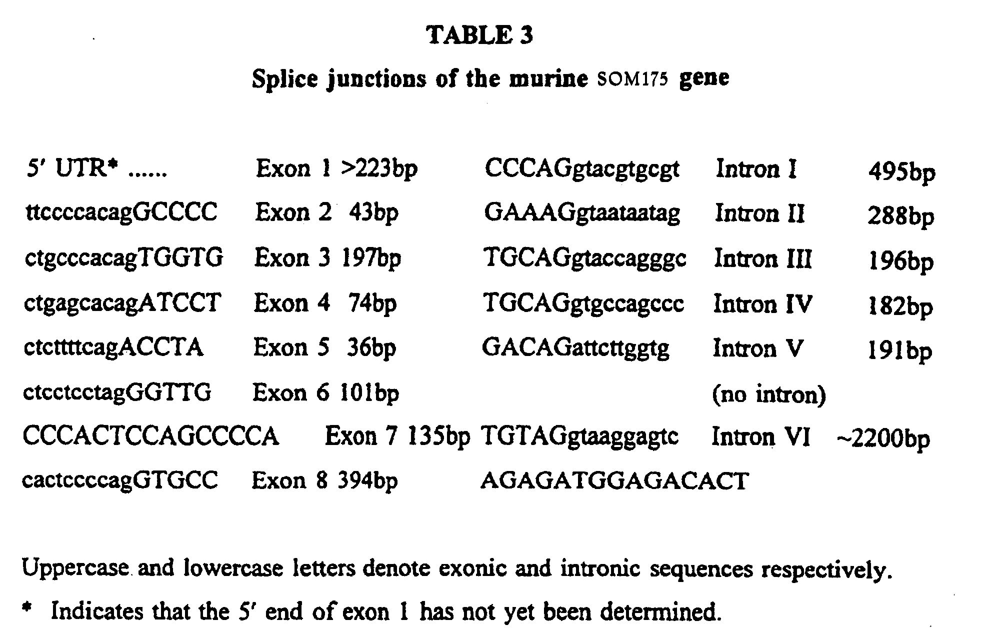

### BIBLIOGRAPHY

Adams MD, Soares MB, Kerlavage AR, Fields C, Venter JC, (1993) *Nature Genet,* **4**, 373-380.
Birnstiel ML, Busslinger M and Strub K (1985) *Cell* **41,** 349-359.
Breier G, Albrecht U, Sterrer S and Risau W (1992) *Development* **114,** 521-532.
Chomczynski P and Sacchi N (1987) *Analyt. Biochem.* **162,** 156-159.
Church G and Gilbert W (1984) *Proc. Natl. Acad Sci. USA* **18,** 1991-1995.
Dagerlind A, Friberg K, Bean AJ and Hokfelt T (1992) *Histochemistry* **98,** 39-49.
Dissen GA, Lara HE, Fabrenbach WH, Costa ME, Ojeda SR, (1994) *Endocrinology* **134,** 1146-1154.
Drinkwater CC, Barker PA, Suter U and Shooter EM (1993) *J. Biol. Chem*., **268,** 23202-23207.
Drinkwater CC, Suter U, Angst C and Shooter EM (1991) *Proc. Roy. Soc. Lond. (Series B),* **246,** 307-313.
Ewton DZ & Florini JR (1980) *Endocrinology,* **106:** 577-583.
Ferrara N & Henzel WJ (1989) *Biochem. Biophys. Res. Commun.* **161,** 851-858.
Folkman J & Shing Y (1992) *J. Biol. Chem.* **267,** 10931-10934.
Gospodarowicz D, Abraham JA & Schilling J (1989) *Proc. Natl. Acad. Sci USA* **86,** 7311-7315.
Gospodarowicz D, Weseman J, Morgan JS & Lindstrom J (1976) *J. Cell Biol.,* **70:** 395-405.
Hagg T, Quon D, Higaki J & Varon S (1992) *Neuron,* **8,** 145-158.
Hefti S (1986) *J. Neurosci,* **6,** 2155-2162.
Hendry IA & Campbell J (1976) *J. Neurocytol*., **5**, 351-360.
Hendry IA, Murphy M, Hilton DJ, Nicola NA & Bartlett PF (1992) *J. Neurosci.* **12,** 3427-3434.
Hunt *et al.,* (1967) *Am. J. Surgery,* **114:** 302-307.
Koch AE, Harlow LA, Haines GK, Amento EP, Unemoti EN, Wong WL, Pope RM,
Ferrara N, (1994) *J. Immunol.* **152,** 4149-4156.
Kromer AF (1987) *Science,* **235,** 214-216.
Larsson C, Weber G, Kvanta E, Lewis C, Janson M, Jones C, Glaser T, Evans G, Nordenskjold M, (1992) *Hum. Genet.* **89,** 187-193.
OZUS Leung DW, Cachianes G, Kuang W-J, Goeddel DV & Ferrara N (1989) *Science* **246**:1306-1309.
Lowe C, Cornish J, Callon K, Martin TJ & Reid IR (1991) *J*. *Bone Mineral Res*., **6**, 1277-1283.
Lowe C, Cornish J, Martin TJ & Reid IR (1991) *Calcif. Tissue Int*., **49,** 394-397. Martinou JC, Martinou I & Kato AC (1992) *Neuron,* **8**, 737-744.
Maruta *et al* (1993) *Growth Factors* **8**: 119-134.
Midy V & Plouet J (1994) *Biochem. Biophys. Res. Commun*., **199**: 380-386.
Miles AA & Miles EM (1952) *J.Physiol. (Lond)* **118**:228-257.
Montesano R, Vassalli JD, Baird A, Guillemin R & Orci, L (1986) *Proc. Natl. Acad*. *Sci USA*, **83,** 7297-7301.
Nurcombe *et al* (1992) *Development* **116**: 1175-1183.
Otto D., Frotscher M & Unsicker K (1989) *J. Neurosci. Res*., **22**, 83-91.
Pepper MS, Ferrara N, Orci L, Montesano R. (1991) *Biochem. Biophys. Res. Commun.* **181,** 902-906).
Rochell JM, Watson ML, Oakey RJ and Seldin MF (1992) *Genomics* **14,** 26-31.
Roth S & Weston J (1967) *Proc. Natl*. *Acad*. *Sci USA*, **58**: 974-980.
Sambrook J, Fritsch EF, Maniatis T, (1989) *Molecular Cloning: A Laboratory Manual - 2nd Ed*. Cold Spring Harbor Laboratory Press, Cold Spring Harbor, N.Y.
Santos MA (1991) *Nucleic Acids Res*. **19,** 5442.
Schilling *et al.,* (1959) *Surgery,* **46:** 702-710.
Senger DR, Van De Water L, Brown LF, Nagy JA, Yeo KT, Yeo TK, Berse B,
Jackman RW, Dvorak AM, Dvorak HF (1993) *Cancer Netastasis Rev*. **12,** 303-324.
Sharkey AM, Chamock-Jones DS, Boocock CA, Brown KD, Smith SK, (1993) *J*. *Reprod*. *Fertil*. **99,** 609-615.
Sunderkotter C, Steinbrink K, Goebeler M, Bhardway R, Sorg E, (1993) *J*. *Leukocyt, Biol*. **55,** 410-422.
Suter U, Angst C, Tien C-L, Drinkwater CC, Lindsay RM and Shooter EM (1992) *J*. *Neurosci*., **12,** 306-318.
Tischer E, Mitchell R, Hartman T, Silva M, Gospodarowicz D, Fiddes JC, & Abraham J (1991) *J. Biol. Chem*. **266**, 11947-11954.
Williams LR, Varon S, Peterson GM, Wictorin K, Fischer W, Bjorklund A & Gage FH (1986) *Proc. Natl. Acad*. *Sci. USA* **83**, 9231-9235.
Yan Z, Weich HA, Bernart W, Breckwoldt M, Neulen J, (1993) *J Clin. Endocrinol. Metab*. **77**, 1723-1725.
Yip NK, Rich KM, Lampe PA & Johnson EM Jr (1984) *J. Neurosci*., **4**, 2986-2992.

## Claims

1. An isolated polypeptide which exhibits at least one of the following properties:
(i) an ability to induce proliferation of vascular endothelial cells;
(ii) an ability to interact with *flt*-1/*flk*-1 family of receptors; and/or
(iii) an ability to induce cell migration, cell survival and/or an increase in intracellular levels of alkaline phosphatase;
wherein said polypeptide comprises:
(a) an amino acid sequence encoded by the nucleotide sequence as set forth in SEQ ID NO:3 or a nucleotide sequence which hybridizes over the full length of SEQ ID NO:3 or its complementary form under high stringency conditions of 0.1-1 x SSC/0.1% w/w SDS at 60°C for 1-3 hours; or
(b) an amino acid sequence as set forth in SEQ ID NO:4 or a truncated form of said polypeptide which exhibits at least one of properties (i)-(iii).

2. An isolated polypeptide according to claim 1 comprising an amino acid sequence encoded by the nucleotide sequence as set forth in SEQ ID NO:3, SEQ ID NO:7 or SEQ ID NO:9.

3. An isolated polypeptide according to claim 1 comprising an amino acid sequence as set forth in SEQ ID NO:4, SEQ ID NO:8 or SEQ ID NO:10.

4. An isolated polypeptide according to any one of claims 1 to 3 wherein said polypeptide is of human origin.

5. An isolated polypeptide according to any one of claims 1 to 4 wherein said polypeptide has the capacity to induce astroglial proliferation.

6. An isolated nucleic acid molecule which encodes the polypeptide according to any preceding claim.

7. An isolated nucleic acid molecule according to claim 6 comprising the nucleotide sequence set forth in SEQ ID NO:3, SEQ ID NO:7 or SEQ ID NO:9.

8. An isolated neutralizing antibody to the polypeptide encoded by the nucleic acid molecule according claim 6 or 7.

9. An isolated neutralizing antibody to the polypeptide according to any one of claims 1 to 5.

10. A method for preparing a polypeptide according to any one of claims 1 to 5, said method comprising expressing a nucleic acid molecule encoding a polypeptide according to any one of claims 1 to 5 in a suitable host grown under conditions effective for the synthesis of said polypeptide.

11. An antisense molecule which hybridises over the full length of a nucleic acid molecule according to claim 6 or 7 or its complement.

12. A composition comprising a polypeptide according to any one of claims 1 to 5 and one or more pharmaceutically acceptable carriers and/or diluents.

13. Use of a polypeptide which exhibits at least one of the following properties:
(i) an ability to induce proliferation of vascular endothelial cells;
(ii) an ability to interact with, *flt*-1/*flk*-1 family of receptors; and/or
(iii) an ability to induce cell migration, cell survival and/or an increase in. intracellular levels of alkaline phosphatase;
wherein said polypeptide comprises:
(a) an amino acid sequence encoded by the nucleotide sequence as set forth in SEQ ID NO: 1, 3, 5, 7, or 9 or a nucleotide sequence capable of hybridizing to SEQ ID NO: 1, 3, 5, 7 or 9 or its complementary form under high stringency conditions of 0.1-1 x SSC/0.1% w/w SDS at 60°C for 1-3 hours; or
(b) an amino acid sequence as set forth in SEQ ID NO: 2, 4, 6, 8 or 10 or an amino acid sequence having at least 70% similarity thereto, or a truncated form of said polypeptide,
for preparation of a medicament for inducing astroglial proliferation in a mammal.

14. Use according to claim 13 wherein the polypeptide comprises an amino acid sequence encoded by the nucleotide sequence as set forth in SEQ ID NO:3, SEQ ID NO:7, or SEQ ID NO:9.

15. Use according to claim 13 wherein the polypeptide comprises an amino acid sequence as set forth in SEQ ID NO:4, SEQ ID NO:8 OR SEQ ID NO: 10.

16. Use according to any one of claims 13 to 15 wherein said polypeptide or the nucleotide sequence encoding it is of human origin.

17. Use of a nucleic acid molecule which encodes a polypeptide as defined in any one of claims 13 to 16 for the preparation of a medicament for inducing astroglial proliferation in a mammal.

18. A host cell comprising a nucleic acid molecule according to claims 6 or 7.

19. A vector comprising a nucleic acid molecule according to claims 6 or 7.

## Patentansprüche

1. Isoliertes Polypeptid, welches wenigstens eine der folgenden Eigenschaften aufweist:
(i) eine Fähigkeit, Proliferation von vaskulären Endothelzellen zu induzieren,
(ii) eine Fähigkeit, mit Rezeptoren der Familie *flt*-1/*flk*-1 in Wechselwirkung zu treten, und/oder
(iii) eine Fähigkeit, Zellmigration, Überleben von Zellen und/oder eine Erhöhung intrazellulärer Mengen an alkalischer Phosphatase zu induzieren,
wobei das Polypeptid folgendes umfaßt:
(a) eine Aminosäuresequenz, welche von der Nukleotidsequenz codiert wird, wie sie in SEQ ID NO:3 angegeben ist, oder eine Nukleotidsequenz, welche über die volle Länge von SEQ ID NO:3 oder dessen komplementäre Form unter hochstringenten Bedingungen von 0,1-1 x SSC/0,1% w/w SDS bei 60°C für 1-3 Stunden hybridisiert, oder
(b) eine Aminosäuresequenz, wie sie in SEQ ID NO:4 angegeben ist, oder eine verkürzte Form dieses Polypeptids, welche wenigstens eine der Eigenschaften (i)-(iii) aufweist.

2. Isoliertes Polypeptid nach Anspruch 1, welches eine Aminosäuresequenz umfaßt, die von der Nukleotidsequenz codiert wird, wie sie in SEQ ID NO:3, SEQ ID NO:7 oder SEQ ID NO:9 angegeben ist.

3. Isoliertes Polypeptid nach Anspruch 1, welches eine Aminosäuresequenz umfaßt, wie sie in SEQ ID NO:4, SEQ ID NO:8 oder SEQ ID NO:10 angegeben ist.

4. Isoliertes Polypeptid nach einem der Ansprüche 1 bis 3, wobei das Polypeptid menschlichen Ursprungs ist.

5. Isoliertes Polypeptid nach einem der Ansprüche 1 bis 4, wobei das Polypeptid die Fähigkeit besitzt, astrogliale Proliferation zu induzieren.

6. Isoliertes Nukleinsäuremolekül, welches das Polypeptid nach einem der vorangegangenen Ansprüche codiert.

7. Isoliertes Nukleinsäuremolekül nach Anspruch 6, welches die Nukleotidsequenz umfaßt, wie sie in SEQ ID NO:3, SEQ ID NO:7 oder SEQ ID NO:9 angegeben ist.

8. Isolierter neutralisierender Antikörper gegen das Polypeptid, welches von dem Nukleinsäuremolekül nach Anspruch 6 oder 7 codiert wird.

9. Isolierter neutralisierender Antikörper gegen das Polypeptid nach einem der Ansprüche 1 bis 5.

10. Verfahren zur Herstellung eines Polypeptids nach einem der Ansprüche 1 bis 5, wobei das Verfahren das Exprimieren eines Nukleinsäuremoleküls, welches ein Polypeptid nach einem der Ansprüche 1 bis 5 codiert, in einem geeigneten Wirt, der unter für die Synthese des Polypeptids wirksamen Bedingungen gezüchtet wird, umfaßt.

11. Antisense-Molekül, welches über die volle Länge eines Nukleinsäuremoleküls nach Anspruch 6 oder 7 oder dessen Komplementäres hybridisiert.

12. Zusammensetzung mit einem Polypeptid nach einem der Ansprüche 1 bis 5 und einem oder mehreren pharmazeutisch verträglichen Trägern und/oder Verdünnungsmitteln.

13. Verwendung eines Polypeptids, welches wenigstens eine der folgenden Eigenschaften aufweist:
(i) eine Fähigkeit, Proliferation von vaskulären Endothelzellen zu induzieren,
(ii) eine Fähigkeit, mit Rezeptoren der Familie *flt*/*flk*-1 in Wechselwirkung zu treten, und/oder
(iil) eine Fähigkeit, Zellmigration, Überleben von Zellen und/oder eine Erhöhung intrazellulärer Mengen an alkalischer Phosphatase zu induzieren,
wobei das Polypeptid folgendes umfaßt:
(a) eine Aminosäuresequenz, welche von der Nukleotidsequenz codiert wird, wie sie in SEQ ID NO:1, 3, 5, 7 oder 9 angegeben ist, oder einer Nukleotidsequenz, die in der Lage ist, mit SEQ ID NO:1, 3, 5, 7 oder 9 oder deren komplementärer Form unter hochstringenten Bedingungen von 0,1-1 x SSC/0,1% w/w SDS bei 60°C für 1-3 Stunden zu hybridisieren, oder
(b) eine Aminosäuresequenz, wie sie in SEQ ID NO:2, 4, 6, 8 oder 10 angegeben ist, oder eine Aminosäuresequenz mit wenigstens 70% Ähnlichkeit dazu oder eine verkürzte Form dieses Polypeptids für die Herstellung eines Medikaments zur Induzierung astroglialer Proliferation in einem Säuger.

14. Verwendung nach Anspruch 13, wobei das Polypeptid eine Aminosäuresequenz umfaßt, die von der Nukleotidsequenz codiert wird, wie sie in SEQ ID NO:3, SEQ ID NO:7 oder SEQ ID NO:9 angegeben ist.

15. Verwendung nach Anspruch 13,.wobei das Polypeptid eine Aminosäuresequenz umfaßt, wie sie in SEQ ID NO:4, SEQ ID NO:8 oder SEQ ID NO:10 angegeben ist.

16. Verwendung nach einem der Ansprüche 13 bis 15, wobei das Polypeptid oder die Nukleotidsequenz, welche dieses codiert, menschlichen Ursprungs ist.

17. Verwendung eines Nukleinsäuremoleküls, welches ein Polypeptid codiert, wie es in einem der Ansprüche 13 bis 16 definiert ist, für die Herstellung eines Medikamentes zur Induzierung astroglialer Proliferation in einem Säuger.

18. Wirtszelle, welche ein Nukleinsäuremolekül nach einem der Ansprüche 6 oder 7 umfaßt.

19. Vektor, welcher ein Nukleinsäuremolekül nach einem der Ansprüche 6 oder 7 umfaßt.

## Revendications

1. Polypeptide isolé qui présente au moins une des propriétés suivantes :
(i) une aptitude à induire la prolifération des cellules endothéliales vasculaires ;
(ii) une aptitude à interagir avec la famille des récepteurs *flt-1*/*flk-1* ; et/ou
(iii) une aptitude à induire la migration de cellules, la survie de cellules et/ou une augmentation des taux intracellulaires de phosphatase alcaline ;
ledit polypeptide comprenant :
(a) une séquence d'amino-acides codée par la séquence de nucléotides représentée dans la SEQ ID n° 3 ou une séquence de nucléotides qui s'hybride sur la totalité de la longueur de la SEQ ID n° 3 ou sa forme complémentaire dans des conditions hautement drastiques consistant en SSC 0,1-1 x/0,1% en poids/poids de SDS à 60°C pendant 1 à 3 heures ; ou
(b) une séquence d'amino-acides représentée dans la SEQ ID n° 4 ou une forme tronquée dudit polypeptide qui présente au moins une des propriétés (i) à (iii).

2. Polypeptide isolé suivant la revendication 1, comprenant une séquence d'amino-acides codée par la séquence de nucléotides représentée dans la SEQ ID n° 3, la SEQ ID n° 7 ou la SEQ ID n° 9.

3. Polypeptide isolé suivant la revendication 1, comprenant une séquence d'amino-acides représentée dans la SEQ ID n° 4, la SEQ ID n° 8 ou la SEQ ID n° 9.

4. Polypeptide isolé suivant l'une quelconque des revendications 1 à 3, ledit polypeptide étant d'origine humaine.

5. Polypeptide isolé suivant l'une quelconque des revendications 1 à 4, ledit polypeptide ayant la capacité à induire la prolifération de l'astroglie.

6. Molécule d'acide nucléique isolée qui code pour le polypeptide suivant l'une quelconque des revendications précédentes.

7. Molécule d'acide nucléique isolée suivant la revendication 6, comprenant la séquence de nucléotides représentée dans la SEQ ID n° 3, la SEQ ID n° 7 ou la SEQ ID n° 9.

8. Anticorps neutralisant isolé contre le polypeptide codé par la molécule d'acide nucléique suivant la revendication 6 ou 7.

9. Anticorps neutralisant isolé contre le polypeptide suivant l'une quelconque des revendications 1 à 5.

10. Procédé pour la préparation d'un polypeptide suivant l'une quelconque des revendications 1 à 5, ledit procédé comprenant l'expression d'une molécule d'acide nucléique codant pour un polypeptide suivant l'une quelconque des revendications 1 à 5 dans un hôte convenable cultivé dans des conditions efficaces pour la synthèse dudit polypeptide.

11. Molécule antisense qui s'hybride sur la totalité de la longueur d'une molécule d'acide nucléique suivant la revendication 6 ou 7 ou son complément.

12. Composition comprenant un polypeptide suivant l'une quelconque des revendications 1 à 5 et un ou plusieurs supports et/ou diluants pharmaceutiquement acceptables.

13. Utilisation d'un polypeptide qui présente au moins une des propriétés suivantes :
(i) une aptitude à induire la prolifération des cellules endothéliales vasculaires ;
(ii) une aptitude à interagir avec la famille des récepteurs *flt-1*/*flk-1* ; et/ou
(iii) une aptitude à induire la migration de cellules, la survie de cellules et/ou une augmentation des taux intracellulaires de phosphatase alcaline ;
ledit polypeptide comprenant :
(a) une séquence d'amino-acides codée par la séquence de nucléotides représentée dans la SEQ ID n° 1, 3, 5, 7 ou 9 ou une séquence de nucléotides capable de s'hybrider la SEQ ID n° 1, 3, 5, 7 ou 9 ou sa forme complémentaire dans des conditions hautement drastiques consistant en SSC 0,1-1 x/0,1% en poids/poids de SDS à 60°C pendant 1 à 3 heures ; ou
(b) une séquence d'amino-acides représentée dans la SEQ ID n° 2, 4, 6, 8 ou 10 ou une séquence d'amino-acides présentant au moins 70% de similarité avec celle-ci, ou une forme tronquée dudit polypeptide,
pour la préparation d'un médicament destiné à induire une prolifération de l'astroglie chez un mammifère.

14. Utilisation suivant la revendication 13, dans laquelle le polypeptide comprend une séquence d'amino-acides codée par la séquence de nucléotides représentée dans la SEQ ID n° 3, la SEQ ID n° 7 ou la SEQ ID n° 9.

15. Utilisation suivant la revendication 13, dans laquelle le polypeptide comprend une séquence d'amino-acides représentée dans la SEQ ID n° 4, la SEQ ID n° 8 ou la SEQ ID n° 10.

16. Utilisation suivant l'une quelconque des revendications 13 à 15, dans laquelle ledit polypeptide ou la séquence de nucléotides codant pour ce polypeptide est d'origine humaine.

17. Utilisation d'une molécule d'acide nucléique qui code pour un polypeptide répondant à la définition suivant l'une quelconque des revendications 13 à 16 pour la préparation d'un médicament destiné à induire la prolifération de l'astroglie chez un mammifère.

18. Cellule hôte comprenant une molécule d'acide nucléique suivant la revendication 6 ou 7.

19. Vecteur comprenant une molécule d'acide nucléique suivant la revendication 6 ou 7.


### SEQUENCE LISTING

(1) GENERAL INFORMATION:
   (i) APPLICANT:
      (countries other than US) AMRAD OPERATIONS PTY. LTD.
      (us only) Hayward, N and Weber, G
   (ii) TITLE OF INVENTION: A NOVEL GROWTH FACTOR AND A GENETIC SEQUENCE ENCODING SAME
   (iii) NUMBER OF SEQUENCES: 14
   (iv) CORRESPONDENCE ADDRESS:
      (A) ADDRESSEE: DAVIES COLLISON CAVE
      (B) STREET: 1 LITTLE COLLINS STREET
      (C) CITY: MELBOURNE
      (D) STATE: VICTORIA
      (E) COUNTRY: AUSTRALIA
      (F) ZIP: 3000
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT INTERNATIONAL
      (B) FILING DATE: 22-FEB-1996
   (vii) PRIOR APPLICATION DATA:
      (A) APPLICATION NUMBER: AU PN1457
      (B) FILING DATE: 02-MAR-1995
      (A) APPLICATION NUMBER: AU PN6647
      (B) FILING DATE: 20-NOV-1995
      (A) APPLICATION NUMBER: AU PN7274
      (B) FILING DATE: 22-DEC-1995
   (viii) ATTORNEY/AGENT INFORMATION:
      (A) NAME: HUGHES DR, E JOHN L
      (C) REFERENCE/DOCKET NUMBER: EJH/EK
   (ix) TELECOMMUNICATION INFORMATION:
      (A) TELEPHONE: +61 3 9254 2777
      (B) TELEFAX: +61 3 9254 2770
(2) INFORMATION FOR SEQ ID NO:1:
   (i) SEQUENCE CHARACTERISTICS:
      (A) LENGTH: 649 base pairs
      (B) TYPE: nucleic acid
      (C) STRANDEDNESS; single
      (D) TOPOLOGY: linear
   (ii) MOLECULE TYPE: DNA
   (ix) FEATURE:
      (A) NAME/KEY: CDS
      (B) LOCATION: 17...589
   (xi) SEQUENCE DESCRIPTION: SEQ ID NO:1:
   TCGGGCCTCC GAAACC ATG AAC TTT CTG CTG TCT TGG GTG CAT TGG AGC         49
                     Met Asn Phe Leu Leu Ser Trp Val His Trp Ser
                       1               5                   10
   CTT GCC TTG CTG CTC TAC CTC CAC CAT GCC AAG TGG TCC CAG GCT GCA       97
   Leu Ala Leu Leu Leu Tyr Leu His His Ala Lys Trp Ser Gln Ala Ala
                15                  20                  25
   CCC ATG GCA GAA GGA GGA GGG CAG AAT CAT CAC GAA GTG GTG AAG TTC      145
   Pro Met Ala Glu Gly Gly Gly Gln Asn His His Glu Val Val Lys Phe
            30                  35                  40
   ATG GAT GTC TAT CAG CGC AGC TAC TGC CAT CCA ATC GAG ACC CTG GTG      193
   Met Asp Val Tyr Gln Arg Ser Tyr Cys His Pro Ile Glu Thr Leu Val
        45                  50                  55
   GAC ATC TTC CAG GAG TAC CCT GAT GAG ATC GAG TAC ATC TTC AAG CCA      241
   Asp Ile Phe Gln Glu Tyr Pro Asp Glu Ile Glu Tyr Ile Phe Lys Pro
    60                  65                  70                  75
   TCC TGT GTG CCC CTG ATG CGA TGC GGG GGC TGC TGC AAT GAC GAG GGC      289
   Ser Cys Val Pro Leu Met Arg Cys Gly Gly Cys Cys Asn Asp Glu Gly
                    80                  85                  90
   CTG GAG TGT GTG CCC ACT GAG GAG TCC AAC ATC ACC ATG CAG ATT ATG      337
   Leu Glu Cys Val Pro Thr Glu Glu Ser Asn Ile Thr Met Gln Ile Met
                    95                 100                 105
   CGG ATC AAA CCT CAC CAA GGC CAG CAC ATA GGA GAG ATG AGC TTC CTA      385
   Arg Ile Lys Pro His Gln Gly Gln His Ile Gly Glu Met Ser Phe Leu
           110                 115                 120
   CAG CAC AAC AAA TGT GAA TGC AGA CCA AAG AAA GAT AGA GCA AGA CAA      433
   Gln His Asn Lys Cys Glu Cys Arg Pro Lys Lys Asp Arg Ala Arg Gln
       125                 130                 135
   GAA AAT CCC TGT GGG CCT TGC TCA GAG CGG AGA AAG CAT TTG TTT GTA      481
   Glu Asn Pro Cys Gly Pro Cys Ser Glu Arg Arg Lys His Leu Phe Val
   140                 145                 150                 155
   CAA GAT CCG CAG ACG TGT AAA TGT TCC TGC AAA AAC ACA GAC TCG CGT      529
   Gln Asp Pro Gln Thr Cys Lys Cys Ser Cys Lys Asn Thr Asp Ser Arg
                   160                 165                 170
   TGC AAG GCG AGG CAG CTT GAG TTA AAC GAA CGT ACT TGC AGA TGT GAC      577
   Cys Lys Ala Arg Gln Leu Glu Leu Asn Glu Arg Thr Cys Arg Cys Asp
               175                 180                 185
   AAG CCG AGG CGG TGAGCCGGGC AGGAGGAAGG AGCCTCCCTC AGCGTTTCGG          629
   Lys Pro Arg Arg
           190
   GAACCAGATC TCTCACCAGG                                                649
(2) INFORMATION FOR SEQ ID NO:2:
   (i) SEQUENCE CHARACTERISTICS:
      (A) LENGTH: 191 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Asn Pro Cys Gly
   130 135 140
   Pro Cys Ser Glu Arg Arg Lys His Leu Phe Val Gln Asp Pro Gln Thr
   145 150 155 160
   Cys Lys Cys Ser Cys Lys Asn Thr Asp Ser Arg Cys Lys Ala Arg Gln
   165 170 175
   Leu Glu Leu Asn Glu Arg Thr Cys Arg Cys Asp Lys Pro Arg Arg
   180 185 190
(2) INFORMATION FOR SEQ ID NO:3:
   (i) SEQUENCE CHARACTERISTICS:
      (A) LENGTH: 1094 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 3..624
   (xi) SEQUENCE DESCRIPTION: SEQ ID NO:3:
   CC ATG AGC CCT CTG CTC CGC CGC CTG CTG CTC GCC GCA CTC CTG CAG          47
      Met Ser Pro Leu Leu Arg Arg Leu Leu Leu Ala Ala Leu Leu Gln
       1               5                  10                  15
   CTG GCC CCC GCC CAG GCC CCT GTC TCC CAG CCT GAT GCC CCT GGC CAC          95
   Leu Ala Pro Ala Gln Ala Pro Val Ser Gln Pro Asp Ala Pro Gly His
                   20                  25                  30
   CAG AGG AAA GTG GTG TCA TGG ATA GAT GTG TAT ACT CGC GCT ACC TGC         143
   Gln Arg Lys Val Val Ser Trp Ile Asp Val Tyr Thr Arg Ala Thr Cys
               35                  40                  45
   CAG CCC CGG GAG GTG GTG GTG CCC TTG ACT GTG GAG CTC ATG GGC ACC   191
   Gln Pro Arg Glu Val Val Val Pro Leu Thr Val Glu Leu Met Gly Thr
           50                  55                  60
   GTG GCC AAA CAG CTG GTG CCC AGC TGC GTG ACT GTG CAG CGC TGT GGT   239
   Val Ala Lys Gln Leu Val Pro Ser Cys Val Thr Val Gln Arg Cys Gly
       65                  70                  75
   GGC TGC TGC CCT GAC GAT GGC CTG GAG TGT GTG CCC ACT GGG CAG CAC   287
   Gly Cys Cys Pro Asp Asp Gly Leu Glu Cys Val Pro Thr Gly Gln His
   80                  85                  90                  95
   CAA GTC CGG ATG CAG ATC CTC ATG ATC CGG TAC CCG AGC AGT CAG CTG   335
   Gln Val Arg Met Gln Ile Leu Met Ile Arg Tyr Pro Ser Ser Gln Leu
                   100                 105                 110
   GGG GAG ATG TCC CTG GAA GAA CAC AGC CAG TGT GAA TGC AGA CCT AAA   383
   Gly Glu Met Ser Leu Glu Glu His Ser Gln Cys Glu Cys Arg Pro Lys
               115                 120                 125
   AAA AAG GAC AGT GCT GTG AAG CCA GAC AGG GCT GCC ACT CCC CAC CAC   431
   Lys Lys Asp Ser Ala Val Lys Pro Asp Arg Ala Ala Thr Pro His His
           130                 135                 140
   CGT CCC CAG CCC CGT TCT GTT CCG GGC TGG GAC TCT GCC CCC GGA GCA   479
   Arg Pro Gln Pro Arg Ser Val Pro Gly Trp Asp Ser Ala Pro Gly Ala
       145                 150                 155
   CCC TCC CCA GCT GAC ATC ACC CAT CCC ACT CCA GCC CCA GGC CCC TCT   527
   Pro Ser Pro Ala Asp Ile Thr His Pro Thr Pro Ala Pro Gly Pro Ser
   160                 165                 170                 175
   GCC CAC GCT GCA CCC AGC ACC ACC AGC GCC CTG ACC CCC GGA CCT GCC   575
   Ala His Ala Ala Pro Ser Thr Thr Ser Ala Leu Thr Pro Gly Pro Ala
                   180                 185                 190
   GCT GCC GCT GCC GAC GCC GCA GCT TCC TCC GTT GCC AAG GGC GGG GCT T   624
   Ala Ala Ala Ala Asp Ala Ala Ala Ser Ser Val Ala Lys Gly Gly Ala
               195                 200                 205
   AGAGCTCAAC CCAGACACCT GCAGGTGCCG GAAGCTGCGA AGGTGACACA TGGCTTTTCA   684
   GACTCAGCAG GGTGACTTGC CTCAGAGGCT ATATCCCAGT GGGGGAACAA AGGGGAGCCT   744
   GGTAAAAAAC AGCCAAGCCC CCAAGACCTC AGCCCAGGCA GAAGCTGCTC TAGGACCTGG   804
   GCCTCTCAGA GGGCTCTTCT GCCATCCCTT GTCTCCCTGA GGCCATCATC AAACAGGACA   864
   GAGTTGGAAG AGGAGACTGG GAGGCAGCAA GAGGGGTCAC ATACCAGCTC AGGGGAGAAT   924
   GGAGTACTGT CTCAGTTTCT AACCACTCTG TGCAAGTAAG CATCTTACAA CTGGCTCTTC   984
   CTCCCCTCAC TAAGAAGACC CAAACCTCTG CATAATGGGA TTTGGGCTTT GGTACAAGAA  1044
   CTGTGACCCC CAACCCTGAT AAAAGAGATG GAAGGAAAAA AAAAAAAAAA            1094
(2) INFORMATION FOR SEQ ID NO:4:
   (i) SEQUENCE CHARACTERISTICS:
      (A) LENGTH: 207 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Ser Pro Leu Leu Arg Arg Leu Leu Leu Ala Ala Leu Leu Gln Leu
    1               5                  10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Arg Ala Ala Thr Pro His His Arg
       130                 135                 140
   Pro Gln Pro Arg Ser Val Pro Gly Trp Asp Ser Ala Pro Gly Ala Pro
   145                 150                 155                 160
   Ser Pro Ala Asp Ile Thr His Pro Thr Pro Ala Pro Gly Pro Ser Ala
                   165                 170                 175
   His Ala Ala Pro Ser Thr Thr Ser Ala Leu Thr Pro Gly Pro Ala Ala
               180                 185                 190
   Ala Ala Ala Asp Ala Ala Ala Ser Ser Val Ala Lys Gly Gly Ala
           195                 200                 205
(2) INFORMATION FOR SEQ ID NO:5:
   (i) SEQUENCE CHARACTERISTICS:
      (A) LENGTH: 993 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 3..566
   (xi) SEQUENCE DESCRIPTION: SEQ ID NO:5:
   CC ATG AGC CCT CTG CTC CGC CGC CTG CTG CTC GCC GCA CTC CTG CAG      47
      Met Ser Pro Leu Leu Arg Arg Leu Leu Leu Ala Ala Leu Leu Gln
        1               5                  10                  15
   CTG GCC CCC GCC CAG GCC CCT GTC TCC CAG CCT GAT GCC CCT GGC CAC      95
   Leu Ala Pro Ala Gln Ala Pro Val Ser Gln Pro Asp Ala Pro Gly His
                    20                  25                  30
   CAG AGG AAA GTG GTG TCA TGG ATA GAT GTG TAT ACT CGC GCT ACC TGC     143
   Gln Arg Lys Val Val Ser Trp Ile Asp Val Tyr Thr Arg Ala Thr Cys
                35                  40                  45
   CAG CCC CGG GAG GTG GTG GTG CCC TTG ACT GTG GAG CTC ATG GGC ACC     191
   Gln Pro Arg Glu Val Val Val Pro Leu Thr Val Glu Leu Met Gly Thr
            50                  55                  60
   GTG GCC AAA CAG CTG GTG CCC AGC TGC GTG ACT GTG CAG CGC TGT GGT     239
   Val Ala Lys Gln Leu Val Pro Ser Cys Val Thr Val Gln Arg Cys Gly
        65                  70                  75
   GGC TGC TGC CCT GAC GAT GGC CTG GAG TGT GTG CCC ACT GGG CAG CAC     287
   Gly Cys Cys Pro Asp Asp Gly Leu Glu Cys Val Pro Thr Gly Gln His
    80                  85                  90                  95
   CAA GTC CGG ATG CAG ATC CTC ATG ATC CGG TAC CCG AGC AGT CAG CTG     335
   Gln Val Arg Met Gln Ile Leu Met Ile Arg Tyr Pro Ser Ser Gln Leu
                   100                 105                 110
   GGG GAG ATG TCC CTG GAA GAA CAC AGC CAG TGT GAA TGC AGA CCT AAA     383
   Gly Glu Met Ser Leu Glu Glu His Ser Gln Cys Glu Cys Arg Pro Lys
               115                 120                 125
   AAA AAG GAC AGT GCT GTG AAG CCA GAT AGC CCC AGG CCC CTC TGC CCA     431
   Lys Lys Asp Ser Ala Val Lys Pro Asp Ser Pro Arg Pro Leu Cys Pro
           130                 135                 140
   CGC TGC ACC CAG CAC CAC CAG CGC CCT GAC CCC CGG ACC TGC CGC TGC      479
   Arg Cys Thr Gln His His Gln Arg Pro Asp Pro Arg Thr Cys Arg Cys
       145                 150                 155
   CGC TGC CGA CGC CGC AGC TTC CTC CGT TGC CAA GGG CGG GGC TTA GAG      527
   Arg Cys Arg Arg Arg Ser Phe Leu Arg Cys Gln Gly Arg Gly Leu Glu
   160                 165                 170                 175
   CTC AAC CCA GAC ACC TGC AGG TGC CGG AAG CTG CGA AGG TGACACATGG       576
   Leu Asn Pro Asp Thr Cys Arg Cys Arg Lys Leu Arg Arg
               180                 185
   CTTTTCAGAC TCAGCAGGGT GACTTGCCTC AGAGGCTATA TCCCAGTGGG GGAACAAAGG    636
   GGAGCCTGGT AAAAAACAGC CAAGCCCCCA AGACCTCAGC CCAGGCAGAA GCTGCTCTAG    696
   GACCTGGGCC TCTCAGAGGG CTCTTCTGCC ATCCCTTGTC TCCCTGAGGC CATCATCAAA    756
   CAGGACAGAG TTGGAAGAGG AGACTGGGAG GCAGCAAGAG GGGTCACATA CCAGCTCAGG    816
   GGAGAATGGA GTACTGTCTC AGTTTCTAAC CACTCTGTGC AAGTAAGCAT CTTACAACTG    876
   GCTCTTCCTC CCCTCACTAA GAAGACCCAA ACCTCTGCAT AATGGGATTT GGGCTTTGGT    936
   ACAAGAACTG TGACCCCCAA CCCTGATAAA AGAGATGGAA GGAAAAAAAA AAAAAAA       993
(2) INFORMATION FOR SEQ ID NO:6:
   (i) SEQUENCE CHARACTERISTICS:
      (A) LENGTH: 188 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Ser Pro Leu Leu Arg Arg Leu Leu Leu Ala Ala Leu Leu Gln Leu
   1               5                   10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                    85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Ser Pro Arg Pro Leu Cys Pro Arg
       130                 135                 140
   Cys Thr Gln His His Gln Arg Pro Asp Pro Arg Thr Cys Arg Cys Arg
   145                 150                 155                 160
   Cys Arg Arg Arg Ser Phe Leu Arg Cys Gln Gly Arg Gly Leu Glu Leu
                   165                 170                 175
   Asn Pro Asp Thr Cys Arg Cys Arg Lys Leu Arg Arg
               180                 185
(2) INFORMATION FOR SEQ ID NO:7:
   (i) SEQUENCE CHARACTERISTICS:
      (A) LENGTH: 858 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 3..431
   (xi) SEQUENCE DESCRIPTION: SEQ ID NO:7:
   CC ATG AGC CCT CTG CTC CGC CGC CTG CTG CTC GCC GCA CTC CTG CAG        47
      Met Ser Pro Leu Leu Arg Arg Leu Leu Leu Ala Ala Leu Leu Gln
       1               5                  10                  15
   CTG GCC CCC GCC CAG GCC CCT GTC TCC CAG CCT GAT GCC CCT GGC CAC       95
   Leu Ala Pro Ala Gln Ala Pro Val Ser Gln Pro Asp Ala Pro Gly His
                   20                  25                  30
   CAG AGG AAA GTG GTG TCA TGG ATA GAT GTG TAT ACT CGC GCT ACC TGC      143
   Gln Arg Lys Val Val Ser Trp Ile Asp Val Tyr Thr Arg Ala Thr Cys
               35                  40                  45
   CAG CCC CGG GAG GTG GTG GTG CCC TTG ACT GTG GAG CTC ATG GGC ACC      191
   Gln Pro Arg Glu Val Val Val Pro Leu Thr Val Glu Leu Met Gly Thr
           50                  55                  60
   GTG GCC AAA CAG CTG GTG CCC AGC TGC GTG ACT GTG CAG CGC TGT GGT      239
   Val Ala Lys Gln Leu Val Pro Ser Cys Val Thr Val Gln Arg Cys Gly
       65                  70                  75
   GGC TGC TGC CCT GAC GAT GGC CTG GAG TGT GTG CCC ACT GGG CAG CAC      287
   Gly Cys Cys Pro Asp Asp Gly Leu Glu Cys Val Pro Thr Gly Gln His
   80                  85                  90                  95
   CAA GTC CGG ATG CAG ATC CTC ATG ATC CGG TAC CCG AGC AGT CAG CTG      335
   Gln Val Arg Met Gln Ile Leu Met Ile Arg Tyr Pro Ser Ser Gln Leu
                   100                 105                 110
   GGG GAG ATG TCC CTG GAA GAA CAC AGC CAG TGT GAA TGC AGA CCT AAA      383
   Gly Glu Met Ser Leu Glu Glu His Ser Gln Cys Glu Cys Arg Pro Lys
               115                 120                 125
   AAA AAG GAC AGT GCT GTG AAG CCA GAT AGG TGC CGG AAG CTG CGA AGG      431
   Lys Lys Asp Ser Ala Val Lys Pro Asp Arg Cys Arg Lys Leu Arg Arg
           130                 135                 140
   TGACACATGG CTTTTCAGAC TCAGCAGGGT GACTTGCCTC AGAGGCTATA TCCCAGTGGG    491
   GGAACAAAGG GGAGCCTGGT AAAAAACAGC CAAGCCCCCA AGACCTCAGC CCAGGCAGAA    551
   GCTGCTCTAG GACCTGGGCC TCTCAGAGGG CTCTTCTGCC ATCCCTTGTC TCCCTGAGGC    611
   CATCATCAAA CAGGACAGAG TTGGAAGAGG AGACTGGGAG GCAGCAAGAG GGGTCACATA    671
   CCAGCTCAGG GGAGAATGGA GTACTGTCTC AGTTTCTAAC CACTCTGTGC AAGTAAGCAT    731
   CTTACAACTG GCTCTTCCTC CCCTCACTAA GAAGACCCAA ACCTCTGCAT AATGGGATTT    791
   GGGCTTTGGT ACAAGAACTG TGACCCCCAA CCCTGATAAA AGAGATGGAA GGAAAAAAAA    851
   AAAAAAA                                                              858
(2) INFORMATION FOR SEQ ID NO:8:
   (i) SEQUENCE CHARACTERISTICS:
      (A) LENGTH: 143 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Ser Pro Leu Leu Arg Arg Leu Leu Leu Ala Ala Leu Leu Gln Leu
    1               5                  10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Arg Cys Arg Lys Leu Arg Arg
       130                 135                 140
(2) INFORMATION FOR SEQ ID NO:9:
   (i) SEQUENCE CHARACTERISTICS:
      (A) LENGTH: 910 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 3..305
   (xi) SEQUENCE DESCRIPTION: SEQ ID NO:9:
   CC ATG AGC CCT CTG CTC CGC CGC CTG CTG CTC GCC GCA CTC CTG CAG        47
      Met Ser Pro Leu Leu Arg Arg Leu Leu Leu Ala Ala Leu Leu Gln
       1               5                  10                  15
   CTG GCC CCC GCC CAG GCC CCT GTC TCC CAG CCT GAT GCC CCT GGC CAC       95
   Leu Ala Pro Ala Gln Ala Pro Val Ser Gln Pro Asp Ala Pro Gly His
                   20                  25                  30
   CAG AGG AAA GTG GTG TCA TGG ATA GAT GTG TAT ACT CGC GCT ACC TGC      143
   Gln Arg Lys Val Val Ser Trp Ile Asp Val Tyr Thr Arg Ala Thr Cys
               35                  40                  45
   CAG CCC CGG GAG GTG GTG GTG CCC TTG ACT GTG GAG CTC ATG GGC ACC      191
   Gln Pro Arg Glu Val Val Val Pro Leu Thr Val Glu Leu Met Gly Thr
           50                  55                  60
   GTG GCC AAA CAG CTG GTG CCC AGC TGC GTG ACT GTG CAG CGC TGT GGT      239
   Val Ala Lys Gln Leu Val Pro Ser Cys Val Thr Val Gln Arg Cys Gly
       65                  70                  75
   GGC TGC TGC CCT GAC GAT GGC CTG GAG TGT GTG CCC ACT GGG CAG CAC      287
   Gly Cys Cys Pro Asp Asp Gly Leu Glu Cys Val Pro Thr Gly Gln His
   80                  85                  90                  95
   CAA GTC CGG ATG CAG ACC TAAAAAAAAG GACAGTGCTG TGAAGCCAGA             335
   Gln Val Arg Met Gln Thr
                   100
   CAGGGCTGCC ACTCCCCACC ACCGTCCCCA GCCCCGTTCT GTTCCGGGCT GGGACTCTGC    395
   CCCCGGAGCA CCCTCCCCAG CTGACATCAC CCATCCCACT CCAGCCCCAG GCCCCTCTGC    455
   CCACGCTGCA CCCAGCACCA CCAGCGCCCT GACCCCCGGA CCTGCCGCTG CCGCTGCCGA    515
   CGCCGCAGCT TCCTCCGTTG CCAAGGGCGG GGCTTAGAGC TCAACCCAGA CACCTGCAGG    575
   TGCCGGAAGC TGCGAAGGTG ACACATGGCT TTTCAGACTC AGCAGGGTGA CTTGCCTCAG    635
   AGGCTATATC CCAGTGGGGA ACAAAGAGGA GCCTGGTAAA AAACAGCCAA GCCCCCAAGA    695
   CCTCAGCCCA GGCAGAAGCT GCTCTAGGAC CTGGGCCTCT CAGAGGGCTC TTCTGCCATC    755
   CCTTGTCTCC CTGAGGCCAT CATCAAACAG GACAGAGTTG GAAGAGGAGA CTGGGAGGCA    815
   GCAAGAGGGG TCACATACCA GCTCAGGGGA GAATGGAGTA CTGTCTCAGT TTCTAACCAC    875
   TCTGTGCAAG TAAGCATCTT ACAACTGGCT CTTCC                               910
(2) INFORMATION FOR SEQ ID NO:10:
   (i) SEQUENCE CHARACTERISTICS:
      (A) LENGTH: 101 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Ser Pro Leu Leu Arg Arg Leu Leu Leu Ala Ala Leu Leu Gln Leu
    1               5                  10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Thr
               100
(2) INFORMATION FOR SEQ ID NO:11:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:11:
   ACCACCACCT CCCTGGGCTG GCATGTGGCA CGTGCATAAA CG                    42
(2) INFORMATION FOR SEQ ID NO:12:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:12:
   AGTTGTTTGA CCACATTGCC CATGAGTTCC ATGCTCAGAG GC 42
(2) INFORMATION FOR SEQ ID NO:13:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:13:
   GATCCTGGGG CTGGAGTGGG ATGGATGATG TCAGCTGG 38
(2) INFORMATION FOR SEQ ID NO:14:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:14:
   GCGGGCAGAG GATCCTGGGG CTGTCTGGCC TCACAGCACT 40